(19) Europäisches Patentamt
European Patent Office
Office européen des brevets

(11) Publication number: **0 492 179 A2**

(12) **EUROPEAN PATENT APPLICATION**

(21) Application number: **91120566.4**

(22) Date of filing: **29.11.91**

(51) Int. Cl.⁵: **C12N 15/12**, C12N 15/00, C07K 15/00, A61K 37/02, C12P 21/02, C12Q 1/68

(30) Priority: **28.12.90 US 634511**
**18.06.91 US 718301**

(43) Date of publication of application:
**01.07.92 Bulletin 92/27**

(84) Designated Contracting States:
**AT BE CH DE DK ES FR GB GR IT LI LU NL SE**

(71) Applicant: **AMERICAN CYANAMID COMPANY**
**1937 West Main Street P.O. Box 60**
**Stamford Connecticut 06904-0060(US)**

(72) Inventor: **Bingham, Brendan William**
**31 Tamarack Court**
**Newtown, Pennsylvania 18940(US)**
Inventor: **Baumbach, William Robert**
**32 Louellen Street**
**Hopewell, New Jersey 08525(US)**
Inventor: **Oldham, Elizabeth Rae**
**1 Leedom Place**
**Newtown, Pennsylvania 18940(US)**

(74) Representative: **Wächtershäuser, Günter, Dr.**
**Tal 29**
**W-8000 München 2(DE)**

(54) **Avian growth hormone receptor and binding protein.**

(57) The present invention relates to novel DNA sequences encoding an avian growth hormone receptor and growth hormone binding protein, as well as the purified proteins per se. The invention also relates to methods for increasing growth of domesticated fowl, such as chickens, turkeys, ducks or geese, by manipulation of the expression of the growth hormone receptor gene.

EP 0 492 179 A2

## BACKGROUND OF THE INVENTION

All vertebrates produce one or more different types of growth factors or growth hormones which are responsible for, among other effects, stimulating protein synthesis, cell division and growth, tissue formation, repair and/or maintenance, and storage or release of particular necessary nutrients into or out of specific cells. Such factors or hormones are most frequently proteins or polypeptides; they share the common feature of being manufactured and released by one type of cell, but exerting their ultimate effects on a different type or target cell. Among some of the better known growth mediators are nerve growth factor, epidermal growth factor, fibroblast growth factor, insulin-like growth factors (somatomedin) and growth hormone (somatotropin).

Each of these factors acts initially by binding to a receptor protein, which may be located either on the surface or in the cytoplasm of the particular factor's target cell. The receptor has a binding site which has a high affinity and specificity for the growth factor or hormone; when the binding between factor and receptor occurs, a sequence of reactions is initiated which in some manner alters the functioning of the target cell. However, not all cells express a receptor protein for a particular growth factor or hormone; moreover, even the specific target cell for a given growth factor or hormone may not express the receptor protein at all times in its life cycle.

Of particular interest is the group of peptides known as growth hormones, in particular, somatotropins, because of the apparently broad range of effects that it has in the developing and adult organism. In all vertebrates, a peptide growth hormone is produced by cells in the anterior pituitary, and released into the circulation. The particular target cells for growth hormone are in the liver; interaction of growth hormone with the receptor proteins on the liver cells causes release from the cells of somatomedins, which in turn function in promoting the growth of bone and muscle tissue. Growth hormones may also act directly on tissues such as muscle and fat by binding to receptors on these tissues and causing local release of somatomedins or as yet other undefined effects. In recent years, exogenous administration of growth hormone has been used to manipulate the ratio of lean muscle to fat in livestock, such as cattle and pigs, in order to produce healthier meat products, as well as to enhance growth efficiency.

Clearly the use of growth hormone has great potential to enhance the efficiency of growth in a broad range of animals which are currently used for food. This potential has not been realized in poultry, however. In chickens, for example, the receptor for growth hormone is not fully functional until the animal is about at least eight to ten weeks of age or later, although growth hormone levels are highest at 1-4 weeks of age at this time (Leung et al., Proc. Soc. Exp. Biol. Med. 184:234-238, 1987); however, most commercial meat chickens are killed at the age of about six weeks. Thus, administration of growth hormone will have little or no effect in accelerating growth or altering the balance of muscle to fat in commercial chickens (Leung et al., Gen. Comp. Endocrinol. 56:389-400, 1984).

Alteration of the expression of the growth hormone receptor in chickens would be potentially useful in permitting earlier action of growth hormone in developing chickens. However, to date, no information has been available on the structure of the growth hormone receptor in any birds. The present invention now provides both the DNA and amino acid sequence of the chicken growth hormone receptor, as well as an insight into the post-transcriptional processing of the mRNA of the receptor. This information provides a basis for altering the natural pattern of GH receptor expression in birds.

## SUMMARY OF THE INVENTION

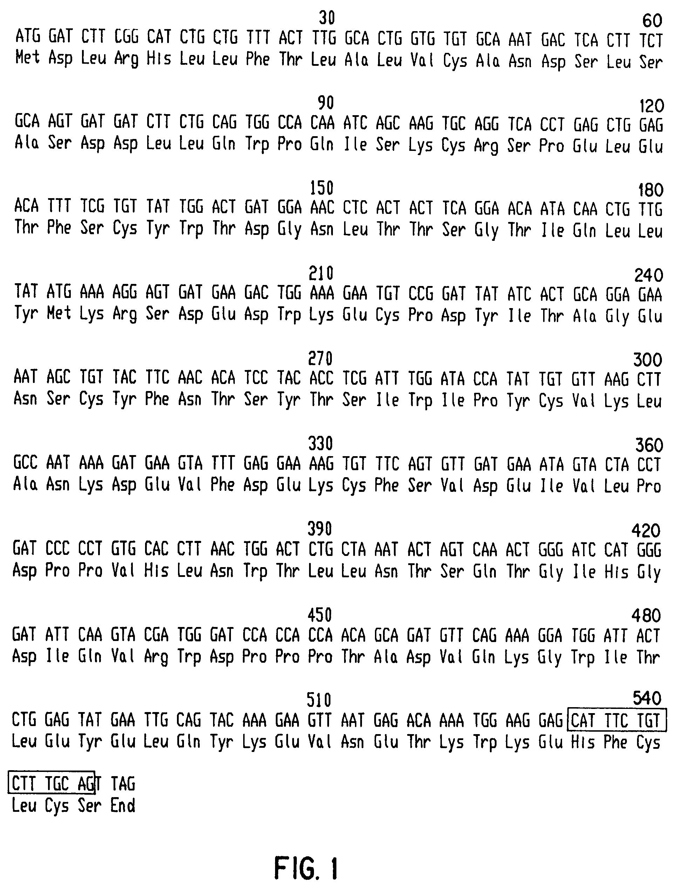

The present invention relates to isolated novel nucleic acid sequences that encodes an avian growth hormone receptor protein. The invention also relates to a novel isolated growth hormone receptor protein, an amino acid sequence of which is also disclosed herein. The invention encompasses not only the nucleic acid and protein sequences depicted in Figure 1 but also encompasses modifications in such sequences which result in a biologically equivalent protein. Data are presented herein which indicate that the early absence of expressed receptor protein is the result of regulation of expression by post-transcriptional cleavage and poly(A) addition of the receptor mRNA. Therefore, modifications in the nucleotide sequence which circumvent alternative cleavage and poly(A) addition are particularly contemplated.

The availability of these sequences also provides a means for altering the expression of the growth hormone receptor in young birds. Thus, the invention also relates to methods for enhancing growth in young birds, particularly chickens. One method of achieving this is the creation of transgenic chickens by insertion into an egg nucleus a nucleic acid construct comprising the nucleotide sequence of the growth hormone receptor, which construct permits early expression of the receptor in the cells of the bird developing therefrom, or subsequent generations of that bird. This can also be achieved by sperm-mediated transfer of the receptor sequence. Alternately, the embryo may be infected by a non-replicating or non-pathogenic virus, such as a retrovirus, which has been genetically manipulated to carry the sequence for the growth hormone receptor. The infection of the embryo by the altered virus will result in the inheritance of the sequence from one cell to another, and ultimately results in somatic expression of the receptor protein in cells derived from infected cells. For purposes of the present specification and claims, the term "transgenic" will be used to apply both to birds which result from introduction of the receptor sequence into the germ line cells, as well as birds which have acquired the gene by somatic cell transformation, in either case, by any of the aforementioned methods of transformation. The invention therefore also encompasses transgenic birds of any species which are capable of expression of the growth hormone receptor prior to the time of its normal expression. For example, for chickens this would be at an age of less than about eight weeks, and preferably less than six weeks, as well as expression vectors comprising the receptor sequence, which vectors are capable of transforming and/or infecting chicken cells so as to permit early expression of the receptor.

Additionally, the availability of a purified receptor gene and receptor protein provides a means for immunization of a host animal with growth hormone receptor fragments. Such immunizations have been shown in U.S. Patent No. 4,857,637, incorporated herein by reference, to act as agonists for growth hormone activity. The present invention thus also relates to therapeutic compositions comprising immunogenic-effective amounts of a chicken growth hormone receptor or fragments thereof in combination with a physiologically acceptable carrier. Successful use of this method requires that the treated animal have growth hormone receptors expressed on cell surfaces.

Moreover the invention also provides a gene and protein sequence for an avian growth hormone binding protein. The binding protein can be used therapeutically to stabilize growth hormone in vivo by prolonging its life in the blood and protecting it from protease degradation. Additionally, binding protein may be useful in reducing levels of growth hormone, either by immunological or transgenic means. In this regard, therapeutic compositions comprising effective amounts of avian growth hormone binding protein, with or without accompanying growth hormone, in combination with a physiologically acceptable carrier, are also useful to the practice of the present invention.

## BRIEF DESCRIPTION OF THE DRAWINGS

Figure 1 (Sequence Listing 1) illustrates the amino acid and nucleotide sequence of a cDNA clone having 17 base pair insertion within the growth hormone receptor sequence producing a frameshift which results in a premature stop codon and a severely truncated protein. The boxed area indicates the region of insertion.

Figure 2 (Sequence Listing 2) illustrates the amino acid and nucleotide sequence of the putative chicken growth hormone binding protein having a single base pair (G) deletion at position 580. The arrow indicates the position of the deletion.

Figure 3 (Sequence Listing 3) illustrates the amino acid and nucleotide sequence of the chicken growth hormone receptor.

Figure 4 shows the mapping of various subclones in relation to the rat growth hormone receptor.

Figure 5 shows the mapping of several different subclones, as well as constructed clones, relative to each other and to the full-length chicken growth hormone receptor.

Figure 6 shows the Southern analysis of one week old chicken liver cDNA library. An aliquot of the cDNA library is sized fractionated on an 0.8% agarose gel, transferred to nytran and probed with a radio-labeled 400 nucleotide Eco RI/Nco I fragment excised from avian GH receptor clone C23.6A. Three species of cDNA are detected, at approximately 4.5 kb, 0.4 kb and 0.5 kb (A). All species correspond in size to those observed in Northern analysis (B); based on data provided in Table 2, infra.

Figure 7 (Sequence Listing 4) shows sequence analysis of clone CH 500.2 (0.5 kb transcript).

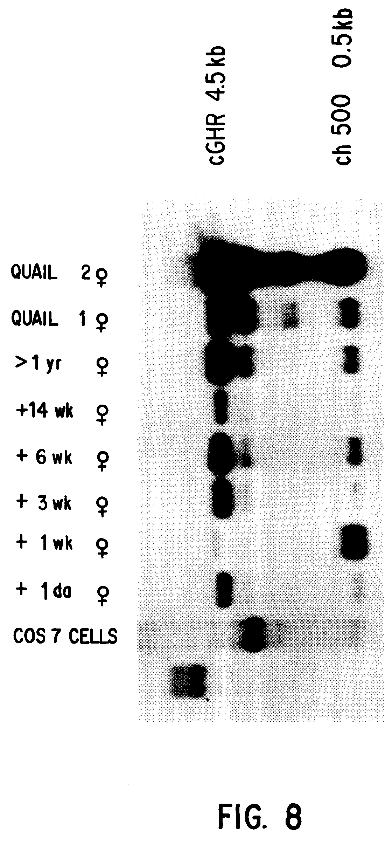

Figure 8 shows Northern analysis of mRNA isolated from domestic and wild avian species, and from COS-7 cells transfected with pSVL-cGHR. Cytoplasmic and poly(A)$^+$ RNA prepared from livers of broiler breeders, quail and COS-7 cells as described. 3 ug of poly(A)$^+$ RNA is size fractionated in a 0.8% agarose/formaldehyde gel, transferred and probed with a radio-labeled 306 nucleotide Hind III/Eco RI fragment excised from pSPORT CH 500.5. Results from this analysis show that wild birds (as represented by quail) possess all three species of mRNA (4.5 kb, 4.0 kb and 0.5 kb) detected in domesticated chickens. In addition a transcript 1.0 kb, which hybridizes to this probe, is also observed in quail.

Figure 9 is a Scatchard plot illustrating binding of growth hormone to chicken growth hormone receptor expressed in COS-7 cells.

## DETAILED DESCRIPTION OF THE INVENTION

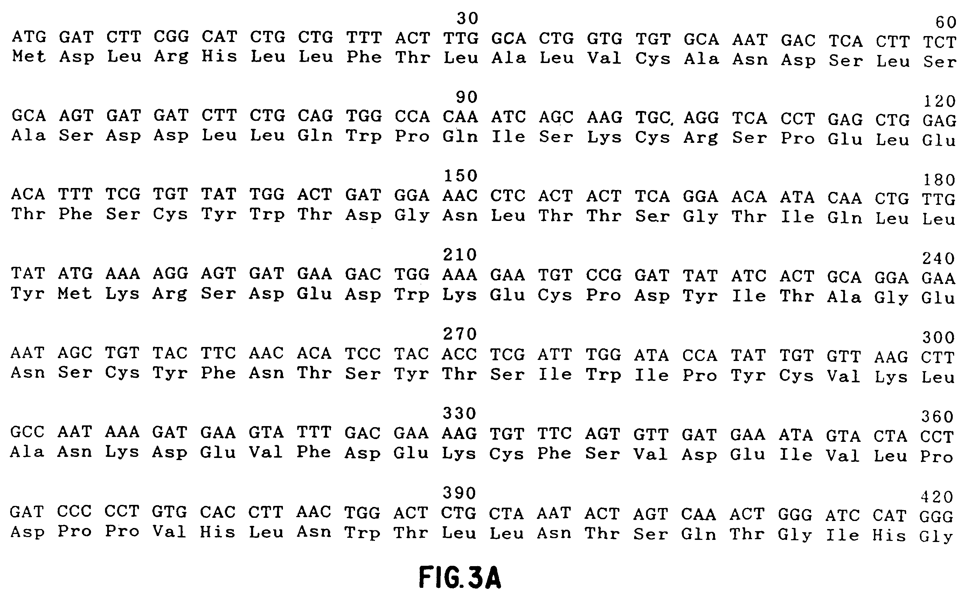

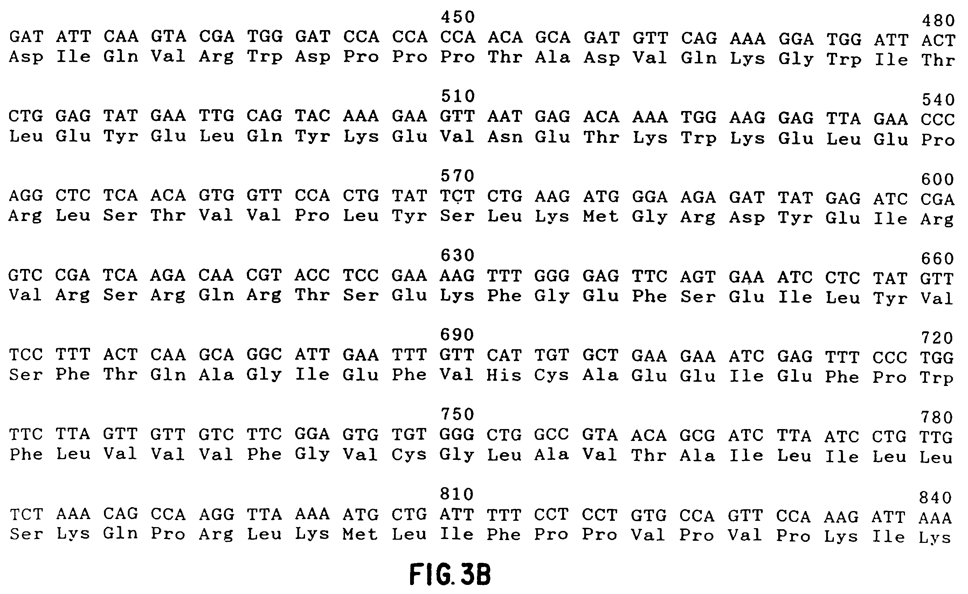

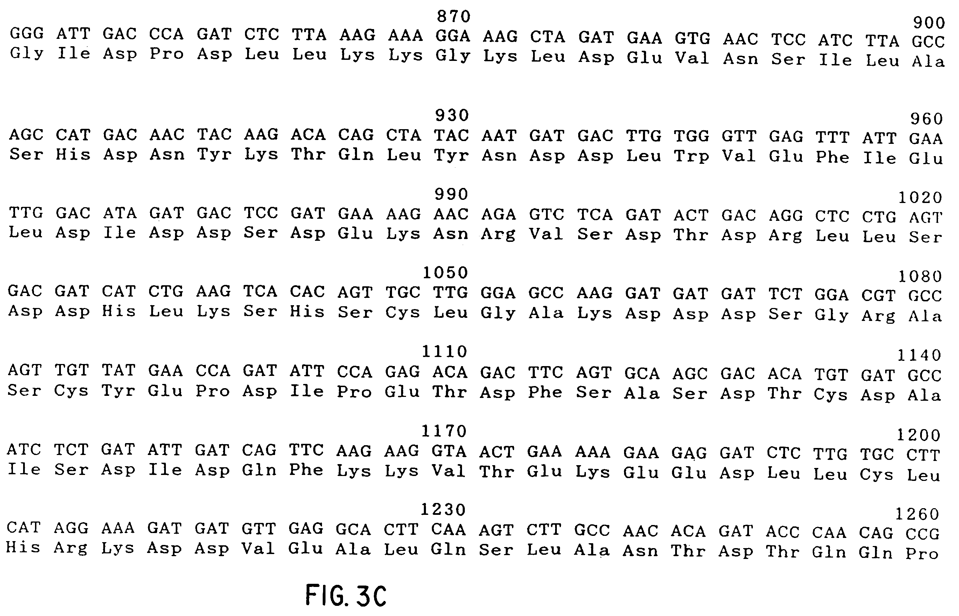

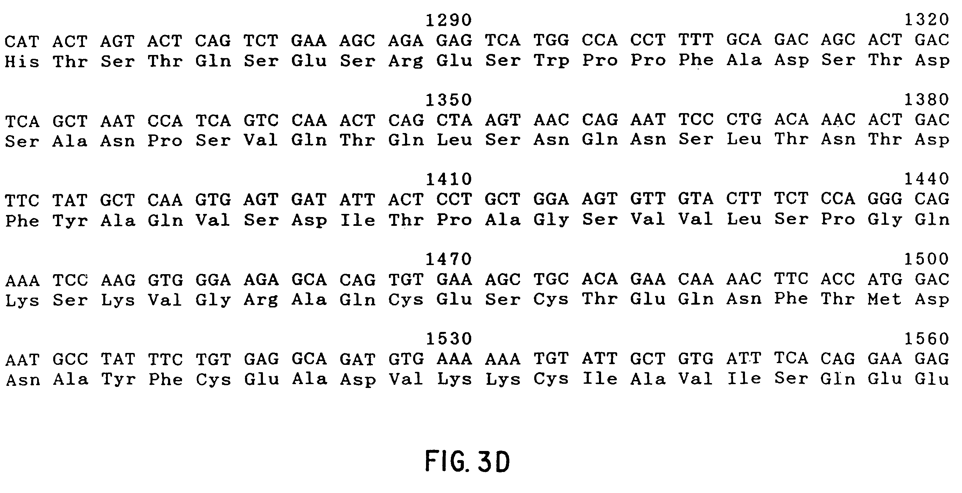

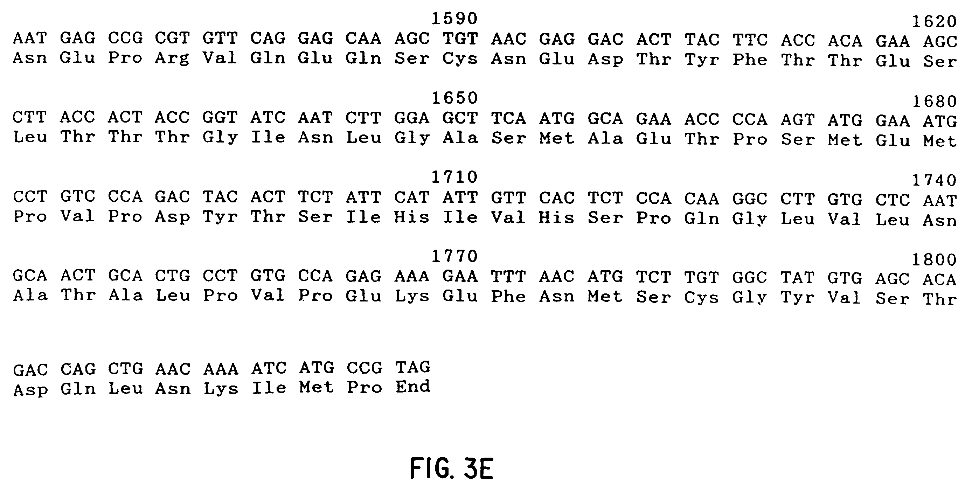

The present invention provides a nucleotide sequence encoding an avian growth hormone receptor protein, as well as the protein produced thereby. The full length receptor DNA is reconstructed from a series of clones isolated from a cDNA library obtained from young broiler chickens. Several classes of clones are combined to create a hybrid full length clone having substantial (i.e., 60% or more) homology with known mammalian growth hormone receptor sequences. A first class of clones, represented by CH8.2F encodes a normal growth hormone receptor to about position 1100. A second class of clones, represented by CH21.9A, contains a frameshift mutation (missing a G relative to the other classes of clones) at position 580; this results in a stop codon about 150 bases later. A third class, represented by CH10.2M, contains a 17 base pair insertion which creates a frameshift relative to the other class of clones; this results in an almost immediate stop codon. A hybrid clone, pCH21.9F containing fragments without the frameshift mutations from either mutantclass possesses a full length sequence of the avian growth hormone receptor. The whole receptor has an amino acid sequence of about 608 amino acids. Both the nucleotide and amino acid sequences are depicted in Figure 3. Comparison with the known rat sequence shows over 60% homology.

In addition to the sequence of the growth hormone receptor, the truncated sequences of CH21.9A and CH10.2M are able to encode a potential growth hormone binding protein, i.e., a soluble circulating form of the GH receptor. Circulating growth hormone-binding proteins have been identified in a number of animals (e.g. Smith et al., Mol. Endocrinol. 3:984-990, 1989; Baumbach et al., Genes & Development 3: 1199-1205, 1989; Leung et al. Nature 330:537-543, 1987; W088/09818). These proteins are typically identical to the GH receptor except for the carboxy terminal portion of the protein. The protein encoded by the sequence noted in CH21.9A contains a sufficient portion of the extracellular domain of the receptor protein so as to reasonably predict that GH binding is a property of the protein.

Northern analysis of cytoplasmic RNA of broiler pullets and cockerels with CH 8.6 as probe initially indicates the presence of a small (about 0.7-0.9 kb) species of RNA. This species is particularly abundant in relatively young birds (see Table 2). A cDNA library based on mRNA from one week old birds yields several clones apparently corresponding to this species. Further analysis indicates that four of these clones have an identical 3'-end structure, which structure is also identical to the first 325 nucleotides of the chicken GH receptor coding region corresponding to mammalian exons 2, 4 and 5. All clones also exhibit the addition of a poly(A) tail after position 325. This position is located 17 bases after the sequence AATAAA (position 304-309), which is the consensus poly(A) addition signal in eukaryotes (Wickens, M., TIBS 15:277-281, 1990). Poly(A) addition is predicted to occur 10 to 30 nucleotides after the AATAAA consensus sequence. In addition, a diffuse GT-rich region ranging from 0 to 10 nucleotides 3' of the poly(A) addition site is the other required element in mammalian and other higher eukaryotic poly(A) addition signals (Proudfoot, N., Cell 65:671-674, 1991). The sequence following the putative cleavage site in the chicken GH receptor is AAAAGTGTTTCAGTGTTG, a motif that fulfills the predicted requirement for the downstream poly(A) addition element. The actual cleavage site may occur after any one of the 4 A's in positions 326-329 without changing the final structure of this message.

Most vertebrate mRNAs are post-transcriptionally processed in a two step process whereby specific cleavage of RNA polymerase II primary transcripts is followed by the addition of a poly(A) tail 50-300 nucleotides long (reviewed by Wickens, TIBS 15:277-281, 1990; Proudfoot, 1991, supra). In some cases, alternative cleavage and poly(A) addition may result in the production of two or more different mRNAs from a single primary message. Such alternative use of poly(A) addition is regulated in a variety of systems, such as in IgM heavy chain ($\mu$) switching from membrane bound to secreted forms (Alt et al., Cell 20:293-301, 1980); the calcitonin/CGRP gene (Leff et al., Cell 48:517-524, 1987); tissue specific isoforms of tropomyosin (Helfman et al., Mol. Cell Biol. 6:3852-3860, 1986), and rat GH receptor and binding protein (Baumbach et al., 1989, supra). In these systems, alternative poly(A) addition is tied to alternative splicing, whereby the inclusion of an exon containing an alternative poly(A) addition signal in the processed mRNA determines the 3' end of the message. It is nevertheless hypothesized that cleavage and poly(A) addition may preceed splicing (Takagaki et al., Genes & Dev. 5:2112-2120, 1990).

The short chicken GH receptor RNA species is highly likely to be the result of cleavage and polyadenylation of the primary GH receptor transcript. The 0.7-0.9 kb estimated size represents the message comprising the poly(A)tail. Further analysis shows the mRNA without the poly(A) region to be about 0.5 kb. It should be noted that this class of transcripts has no stop codon, and is thus unlikely to be efficiently translated. If translation did occur, multiple lysine residues would result from reading of the poly-(A) tail of the truncated message.

Since, in the case of the chicken GH receptor message, a polyadenylation signal resides within the coding region of the full length receptor, this signal is either chosen or overlooked in a regulated manner throughout development and among tissues in the chicken. A phenomenon whereby retroviruses specifically overlook polyadenylation signals near the 5' end of mRNAs has been explained in hepatitis B viruses by the existence of specific sequences 5' of the canonical AATAAA hexanucleotide (Russnak and Ganem, Genes & Dev. 4:674-676, 1990); in HIV, the GT-rich region found 3' of the AATAAA determines that promotor proximity occludes the use of that upstream site (Weichs an der Glon et al., Genes & Dev. 5:244-253, 1991). A more analogous system is the differential use of poly(A) signals in late versus early stage adenovirus infection. Here, poly(A) site selection appears to occur on a first come, first served basis at early times after infection, whereas two sites are equally used at late times (Falck-Pedersen and Logan, J. Virol. 63:532-541, 1989). Sequences upstream of the AATAAA site were shown to influence poly(A) site selection in response to presumed cellular and/or viral encoded factors in this system (DeZazzo and Imperiale, Mol. Cell Biol. 9:4951-4961, 1989). The choice of poly(A) addition signals in the chicken GH receptor gene strongly influences the levels of functional GH receptor mRNA in the cell. The mechanism whereby this choice occurs may involve a combination of those demonstrated in these various systems, but is clearly unique in its function and regulation.

This regulation of GH receptor mRNA, and the subsequent ability of cells to respond to GH, may have profound effects on growth and production in poultry. The data presented herein strongly suggest that alternative polyadenylation negatively regulates GH receptor levels in avian species. Genetic alteration of the canonical poly(A) addition signal AATAAA, would eliminate this form of control. Thus, the present invention particularly contemplates GH receptor nucleotide sequences in which the AATAAA sequence is replaced by a different sequence encoding the same amino acids (Asn, Lys). Examples of such sequences include AACAAG, AATAAG, and AACAAA. Alteration in vivo can be accomplished using artificial GH receptor genes, such as in transgenic or somatically expressing animals, or through the replacement of the endogenous sequences using homologous recombination.

Alternatively, altering the degree to which the alternative poly(A) addition site is chosen in vivo can also provide a means for increasing GH receptor levels. Thus, screening systems, such as the one described in Example 15, infra, can be developed which would, by observation of the relative production of the .5 and 4.5 kb species of RNA, identify compounds that interfere with or alter the normal pattern of utilization of poly(A) addition sites. This could be achieved, for example, by preventing or reducing the use of the poly(A) site within the coding region, or alternately, by favoring the utilization of an upstream poly(A) site. Such compounds can then be administered to young chickens to increase expression of a functional receptor at an early age.

An isolated gene encoding the growth hormone receptor can be used to create expression vectors which in turn are used to transform host cells, conferring the ability to express the receptor protein on the cell surface and thus to permit growth hormone binding. The ability to transform chicken cells at an early stage of development provides a heretofore unavailable basis for successful application of a growth hormone program since, normally, the receptor is not expressed until 8-10 weeks of age, long after the time at which most meat chickens are killed.

Transformation with the receptor gene can be achieved without vectors by, for example, microinjection of the nucleic acid into an egg nucleus. However, in a preferred embodiment, the gene is placed into a vector which is capable of in turn integrating the gene into the genome of the host cell. One way of achieving this is by sperm-mediated transfer of the sequence into an egg nucleus. Useful constructs can be made which incorporate tissue-specific promoters, such as those for liver, muscle or fat, which tissues are the preferred targets for receptor expression. A viral vector, particularly a retroviral vector, can also be used to achieve host cell transformation. There are a number of avian-associated retroviruses, such as Rous Sarcoma virus, Rous-associated virus, spleen necrosis virus, leukosis viruses, and reticuloendotheliosis viruses. Use of retroviral vectors for transfer of genes into bird embryos has been widely reported (Bandyopadhiyay and Temin, Mol. Cell Biol. 4:743-748, and 749-754, 1984; Salter et al., Virology 157:236-240, 1987; Shuman and Shoffner, Poultry Science 65:1437-1444, 1986; Salter et al., Poultry Science 65:1445-1458, 1986; Hughes et al., Poultry Science 65:1459-1467, 1986). The retrovirus employed is preferably nonpathogenic or replication defective i.e., the viral genome is modified so as to prevent the virus from replicating, but to still permit the virus to infect host cells. Such replication defective viruses have been described, for example in Miller and Buttimore (Mol. Cell. Biol. 6:2895-29020, 1986) and Stoker and Bissell (J. Virol. 62:1008-1015, 1988). Expansion of a replication defective virus population is sometimes achieved by the use of helper cells which complement the defect in the vector, permitting replication within the helper cells, from which the virus stock is ultimately produced (Hu et al., Virology 159:446-449, 1987; Watanabe et al., Mol. Cell. Biol. 3:2241-2249, 1983). The viral stocks, or virus-producing helper cells, are then used to inoculate either an egg nucleus or early embryo. Infection may be either of the germ line of the embryo (see, e.g., Bosselman et al., Science 243:533-535, 1989) or of somatic stem cells (Bosselman et al., J. Virol, 63:2680-2689, 1989). Successful infection will result in the expression of the receptor protein on the surface of infected cells, and incorporation of the modified retroviral vector into the genome of these cells. The transgenic bird which develops from infected cells will thus be capable of responding to endogenous growth hormone, and/or administration of exogeneous growth hormone, at a much earlier age than non-treated birds, for example, in chickens preferably before six to eight weeks of age, and most preferably at the time of hatching.

In addition to its use in transforming chicken cells, the present growth hormone receptor gene may also be useful in transforming cells of other bird species of interest, such as turkey, goose, quail or duck. It is a reasonable expectation that receptor among birds may be interchangeable. Moreover, the DNA sequence encoding the chicken receptor, or portions thereof, provides the basis for identifying the specific receptor gene in other bird species. A relatively high degree of homology exists between the growth hormone receptor gene of mammals and the chicken. Homology would be expected to be even higher among avian species. Therefore, detectably labeled chicken growth hormone receptor nucleic acid can be used to probe genomic libraries of other poultry species, in order to facilitate isolation of other avian growth hormone receptor genes. Thus, the present invention also encompasses all avian nucleic acid sequences which hybridize with the chicken growth hormone receptor gene, under standard high stringency conditions, such as those described in Maniatis et al. (Molecular Cloning. A Laboratory Manual. Cold Spring Harbor Laboratory, Cold Spring Harbor, N.Y., 1989) which nucleic acid encodes a receptor protein capable of binding an avian growth hormone.

The isolated receptor protein itself is useful in setting up screening assays to identify compounds that mimic the activity of the avian growth hormone. For example, a microorganism such as E. coli can produce a truncated form of the receptor protein. The recombinant protein is immobilized with a monclonal antibody that does not interfere with growth hormone binding. This system can be used in an assay whereby specific compounds are mixtures, such as fermentation broths, are tested for the ability to compete with radiolabelled growth hormone for binding to growth hormone receptor. Also, the protein is useful in the production of antibodies, both polyclonal and monoclonal, to the growth hormone receptor. In this regard, attention is drawn to U.S. Patent No. 4, 857,637, the contents of which are incorporated herein by reference, wherein it is noted that growth hormone receptor derivatives may be used to immunize a host against a receptor with the effect of inducing agonist polyclonal antibodies capable of enhancing native receptor activity. Thus, the present invention also provides a therapeutic composition comprising the growth hormone receptor, or immunogenic fragment thereof, in combination with a physiologically acceptable carrier.

The invention also provides a nucleic acid sequence encoding a growth hormone binding protein, and the binding protein per se. As noted above, one of the isolated clones possesses a truncated receptor protein sequence, which sequence is believed to represent the gene encoding the growth hormone binding protein. The putative binding protein sequence is provided in Figure 3. The sequence corresponds with the receptor sequence up to nucleotide 580, wherein there is a deletion of a guanine residue, resulting in a frameshift mutation that produces a stop codon at nucleotide 724-726. However, a substantial segment of the sequence encoding the extracellular portion of the receptor protein is present, indicating the presence of binding ability. Growth hormone binding proteins have been suggested as playing a role in stabilizing growth hormone in vivo, by protecting it from the action of proteases, thereby increasing its life and efficacy. The binding protein can therefore be used therapeutically either as a sole active agent, or with exogenous growth hormone, in combination with a physiologically acceptable carrier. Such compositions may be used in treating animals in need of enhancement of growth hormone activity.

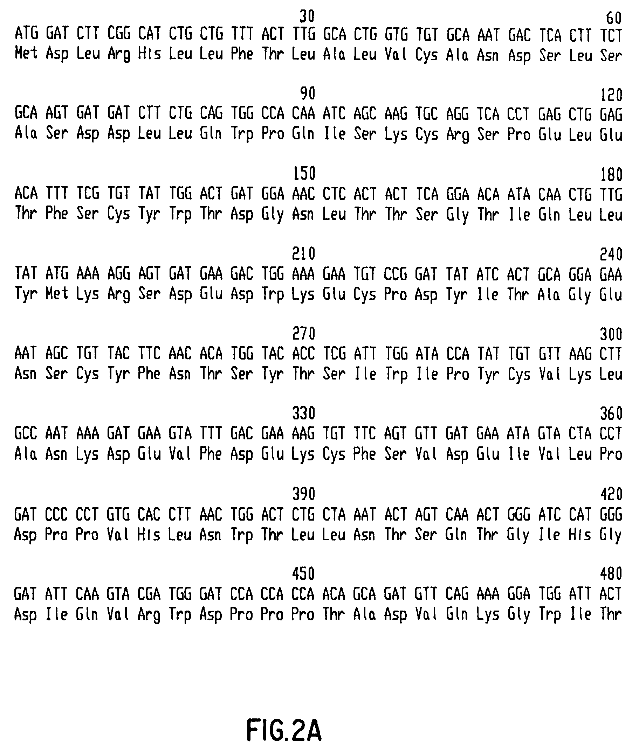

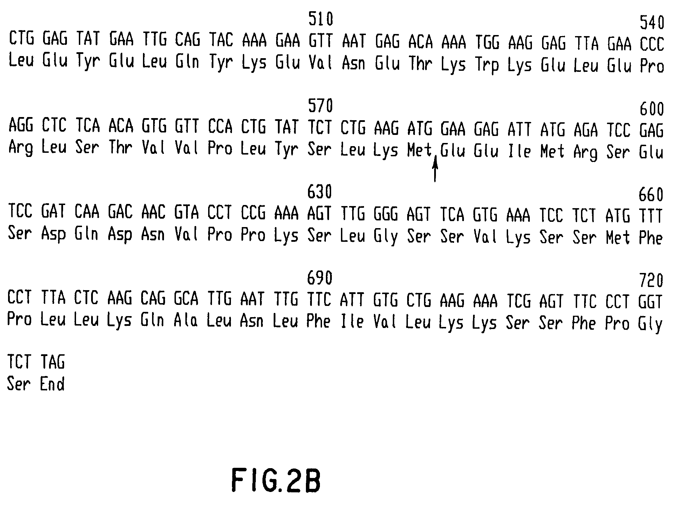

Exemplary nucleotide and amino acid sequences of the various encoded proteins have been provided in Figures 1, 2 and 3. However, it will be understood that the invention is not limited solely to those sequences, but also encompasses biologically equivalent sequences. Modifications to the sequence, such as deletions, insertions, or substitutions in the sequence which produce silent changes in the resulting protein molecule are also contemplated. For example, alteration in the gene sequence which reflect the degeneracy of the genetic code, or which result in the production of a chemically equivalent amino acid at a given site, are contemplated; thus, a codon for the amino acid alanine, a hydrophobic amino acid, may be substituted by a codon encoding another less hydrophobic residue, such as glycine, or a more hydrophobic residue, such as valine, leucine, or isoleucine. Similarly, changes which result in substitution of one negatively charged residue for another, such as aspartic acid for glutamic acid, or one positively charged residue for another, such as lysine for arginine, can also be expected to produce a biologically equivalent product. Nucleotide changes which result in alteration of the N-terminal and C-terminal portions of the protein molecule would also not be expected to alter the activity of the protein. It may also be desirable to eliminate one or more of the cysteines present in the sequence, as the presence of cysteines may result in the undesirable formation of multimers when the protein is produced recombinantly, thereby complicating the purification and crystallization processes. Each of the proposed modifications is well within the routine skill in the art, as is determination of retention of biological activity of the encoded products. Therefore, where the phrases "avian growth hormone receptor nucleotide sequence or gene", "avian growth hormone receptor protein" "avian growth hormone binding protein nucleotide sequence or gene" or "avian growth hormone binding protein" are used in either the specification or the claims, each will be understood to encompass all such modifications and variations which result in the production of a biologically equivalent protein. In particular, the invention contemplates those DNA sequences which are sufficiently duplicative of the sequence of Figure 1 so as to permit hybridization therewith under standard high stringency southern hybridization conditions, such as those described in Maniatis et al., (Molecular cloning. A Laboratory Manual. Cold Spring Harbor Laboratory, 1989), as well as the biologically active proteins produced thereby.

In addition to full length genes and proteins the invention encompasses biologically active fragments of each. By "biologically active" is meant a protein fragment which qualitatively retains the binding activity of the larger protein, or, in the case of a nucleotide sequence, which encodes such a protein fragment. It also refers, for purposes of antibody production, to fragments which are capable of eliciting production of antibodies capable of binding to the receptor and/or binding protein.

The proteins in question can readily be prepared by recombinant methods in a variety of host cells, with or without the use of an expression vector. For example, transformation of an egg nucleus or any appropriate host cell can be achieved directly by, for example, microinjection or electroporation, whereby the nucleic acid is inserted directly into the host cell. Alternately, however, the nucleic acid sequence is incorporated into a vector appropriate to the host so as to achieve expression in the host cell line. For purposes of producing a transgenic bird expressing the receptor protein, the ultimate host cell is avian, and, as already noted, a preferred vector is a retrovirus. However, for research purposes, e.g., for production of protein to be used in antibody production, for observing receptor binding activity of a compound of interest, or for creating therapeutic compositions, it may be desirable to express the receptor protein or binding protein in a non-avian cell line. It is therefore contemplated that the proteins may be produced in any appropriate prokaryotic or eukaryotic cell line, using any expression vector appropriate for the chosen cell line. Examples of suitable eukaryotic cells include mammalian cells, plant cells, yeast cells, and insect cells. Suitable prokaryotic hosts include Escherichia coli and Bacillus subtilis.

Suitable expression vectors are selected based upon the choice of host cell. Numerous vectors suitable for use in transforming bacterial calls are well known. For example, plasmids and bacteriophages, such as λ phage, are the most commonly used vectors for bacterial hosts, and for E.. coli in particular. In both mammalian and insect cells, virus vectors are frequently used to obtain expression of exogenous DNA. In particular, mammalian cells are commonly transformed with SV40 or polyoma virus; and insect cells in culture may be transformed with baculovirus expression vectors. Yeast vector systems include yeast centromere plasmids, yeast episomal plasmids and yeast integrating plasmids. The invention encompasses any and all host cells transformed by the claimed genes, as well as expression vectors used to achieve this transformation.

The invention is further illustrated by the following non-limiting examples.

## EXAMPLES

### 1. Screening and Cloning

cDNA's are isolated by screening a chicken cDNA library prepared from livers of seven week-old male broiler-breeders. The library is purchased from Stratagene (La Jolla, California). First strand synthesis is oligo-dT primed. cDNA inserts are prepared with Eco RI linkers and ligated into the Eco RI cloning site of the phage vector Lambda Zap (Stratagene). The library consists of 2.0 x $10^6$ independent clones of which 95% are recombinant. The average insert size is 1.0 kb.

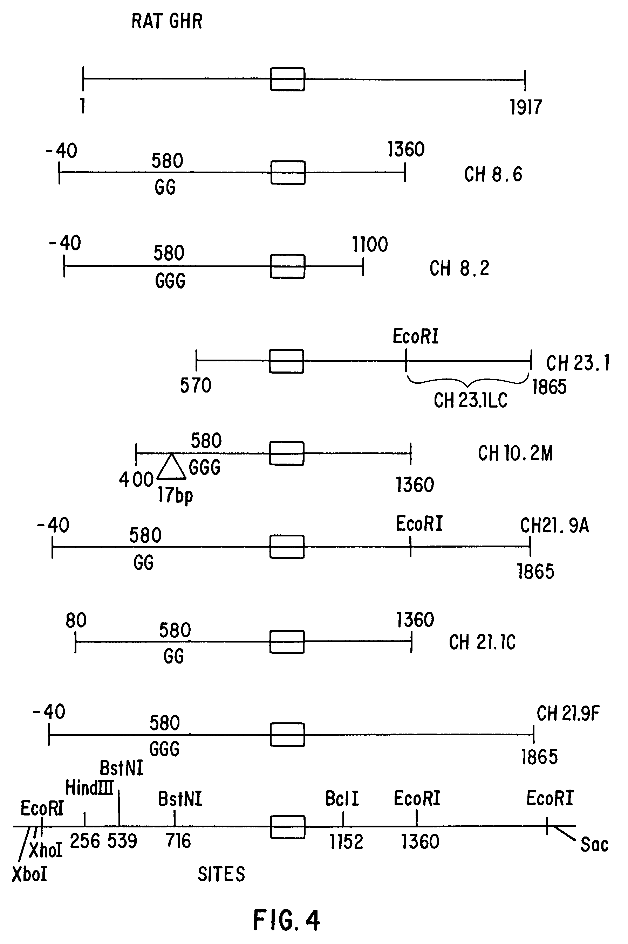

Six primary plates of the library are prepared at a density of $10^5$ clones per 150 mm plate. Nitrocellulose plaque lifts are taken from each plate. A gel purified insert fragment of subclone pS1.1G is $^{32}P$ labeled using random hexamers and DNA polymerase I (Klenow fragment). The pS1.1G insert contains bases 126-777 of the ovine growth hormone receptor extracellular domain. The filters are prehybridized in Church buffer (7% w/v sodium dodecyl sulfate. 0.5% w/v bovine serum albumin, 0.25M $Na_2HPO_4$ pH 7.2, 1mM EDTA) at 55°C for 30 minutes. Hybridization is overnight in Church buffer at 55°C in the presence of the pS1.1G probe. Filters are washed four times, 10 minutes each, in 2 x SSC (1 x SSC is 0.15M NaCl, 0.15M sodium citrate, pH 7.0), 0.2% SDS (sodium dodecyl sulfate) at 45°C. Filters are exposed to film with intensifying screens. These low stringency conditions produce several positive signals per filter. Twelve positives are chosen for secondary screening. Secondary screening is performed by plating primary positives at low density (less than 1000 clones per 85mm plate), lifting filters, prehybridizing for 30 minutes in Church buffer at 55°C, and hybridizing overnight in Church buffer at 55°C with a $^{32}$p-labeled probe. This time the probe is the insert fragment of subclone pRat 1-20, consisting of nucleotides 1-790 of rat growth hormone binding protein; labeling is performed using random hexamers and DNA polymerase I (Klenow fragment). Filters are washed four times, ten minutes each, in 2 x SSC, 0.2% SDS at 48°C. Tertiary screening is according to the same procedure as secondary screening, except that plating is at an even lower density (less than 200 clones per plate). Three of the twelve clones retest through this secondary and tertiary screening. The inserts of these three phage are removed by cleavage with Eco RI, gel purified, and subcloned into the plasmid vector pGem 3zf(-). Sequence analysis of plasmid subclones reveals that two of the three clones (CH8.2 and CH10.2) have a substantial (approximately 60%) sequence similarity to known mammalian growth hormone receptors. The third clone (CH11.1) shows no convincing similarity to known growth hormone receptors and is discarded. Figure 4 shows the mapping of several subclones relative to the growth hormone receptor of rat.

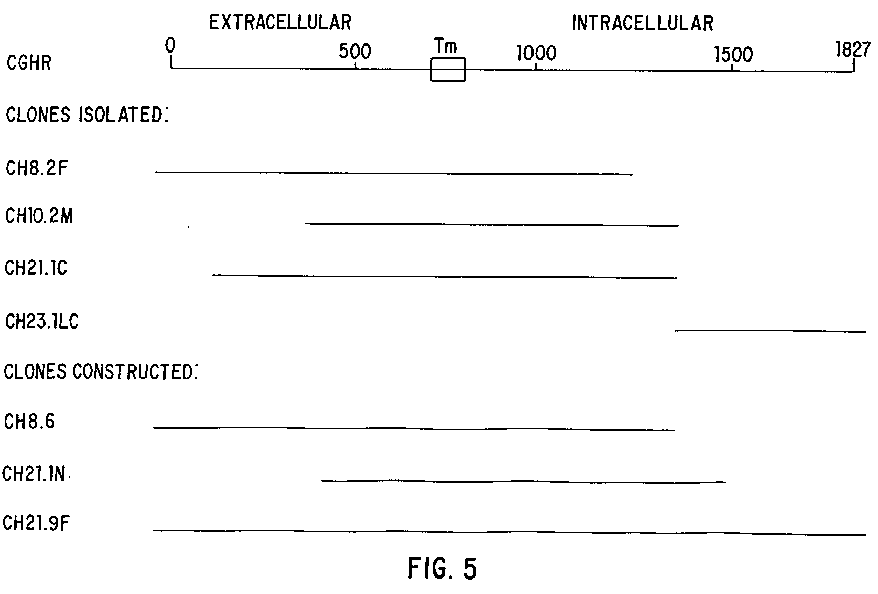

The insert fragment is excised from pCH10.2M by restriction with Eco RI, isolated by gel purification and is $^{32}$p labelled using random hexanucleotides and DNA polymerase I (Klenow fragment). Twelve more primary plates are prepared for screening. Plating is at a density of about $10^5$ clones per plate. Nitrocellulose filters are lifted from the plates. The filters are prehybridized at 62°C in Church buffer. Hybridization is overnight in Church buffer at 62° in the presence of the radiolabeled pCH10.2M probe. High stringency filter washes are performed (four washes, ten minutes each, 0.2 X SSC, 0.2% SDS at 65°C). Several positive clones appear per plate. Twelve are chosen for secondary screening. Secondary and tertiary screening are under the same prehybridization and hybridization conditions as the high stringency primary screening described above. Seven of the twelve clones retest through the secondary and tertiary screening. Eco RI inserts are excised from the phage clones and subcloned into pGem 3zf(-). Restriction mapping is performed and initial sequence information gathered for these subclones. Two subclones (pCH21.1C and pCH23.1LC) are chosen for extensive sequence analysis. Figure 5 shows the location of these subclones relative to pCH8.2F, pCH10.2M, and the full length chicken growth hormone receptor.

2. Sequence Analysis

All sequencing is done using the Sequenase (United States Biochemical) system for double stranded DNA. All sequence reactions use $^{35}$S dATP as the radioactive label. All reactions are run on 6% polyacrylamide gels. Typically, 300 bases of sequence can be read from each reaction.

Each plasmid subclone is sequenced from each end using primers that anneal outside the pGEM 3zf (-) cloning polylinker (e. g., the Sp6, T7, and pUC M13 forward primers). In addition, synthetic oligonucleotides are designed, based on sequence read from the ends of subclones. These synthetic oligonucleotides are used as sequencing primers. The nucleotide sequence and annealing sites of the synthetic oligonucleotides are listed in Table 1.

**TABLE 1**

**Synthetic Oligonucleotides and Annealing Sites**

| | | | | |
|---|---|---|---|---|
| CH1 | 5'-dTTCTTTCCAGTCTTCATCAC-3' | 213 | --> | 194 |
| CH2 | 5'-dTGTCCGGATTATATCACTGC-3' | 214 | --> | 233 |
| CH3 | 5'-dATTAACTTCTTTGTACTGCA-3' | 573 | --> | 554 |
| CH4 | 5'-dGAGACAAAATGGAAGGAGCA-3' | 574 | --> | 593 |
| CH5 | 5'-dACAGGAGGAAAAATCAGCAT-3' | 821 | --> | 802 |
| CH6 | 5'-dCCTGTGCCAGTTCCAAAGAT-3' | 817 | --> | 836 |
| CH7 | 5'-dCTGGTTCATAACAACTGGCA-3' | 1096 | --> | 1077 |
| CH8 | 5'-dGATATTCCAGAGACAGACTT-3' | 1096 | --> | 1115 |
| CH9 | 5'-dAGGCAGATGTGAAAAAATGT-3' | 1517 | --> | 1536 |
| CH10 | 5'-dTCCATACTTGGGGTTTCTGC-3' | 1676 | --> | 1657 |

3. Construction of a Full-Length Avian Growth Hormone Receptor Clone

From the sequences of isolated clones, a full length avian growth hormone receptor cDNA is constructed. The final construct is based on analysis of three major classes of cDNA clones. However, no single cDNA clone contains the full length receptor sequence. The first class is represented by clone CH8.2F which contains the normal chicken growth hormone receptor sequence from the start site (-40) to about base 1100 and 23.1, which contains the normal receptor sequence from about position 570 to 1865. The second class is represented by CH21.1G an original isolated clone, and CH21.9A, a 2 kb Eco RI fragment which is assembled from two shorter cDNA clones, CH8.6 and CH23.1. Each of 21.1C, CH8.6 and CH21.9A contain a frameshift (missing a G relative to the other class of clones) at position 580, resulting in a stop codon about 150 bases later. Both CH8.6 and CH21.9A potentially encode a chicken growth hormone binding protein of about 240 amino acids. The third class is represented by CH10.2M, which contains a 17 base pair insertion relative to the other class of clones. This insertion creates a frameshift that results in a stop codon almost immediately. From among these clones, a hybrid clone encoding a full length receptor analogous to that seen in mammals is engineered.

To construct a functional full length receptor, a vector is created in pGEM3Zf(+), containing the 5' end of CH8.2F from -40 (EcoRI) to 414 (NooI) and the 3' end of CH23.1LC (EcoRI; 1360-1865). CH23.1LC is an EcoRI fragment of the larger clone 23.1 (see Figure 4). Into this vector is cloned simultaneously the NcoI to BglII (414 to 852) portion of CH21.1G. The resulting clone, CH21.9F (pGEM-cGHR), contains a full length chicken growth hormone receptor sequence, and is deposited with the American Type culture collection as Accession No. 68499. This clone is used for expression in eukaryotic systems. This clone encodes a protein that maintains a significant homology with rat growth hormone receptor throughout its length, and also displays increased homology with rat in regions found to be conserved among species.

Similarly, clones are constructed in connection with the putative binding protein sequence. Clone 21.1C extends from positions 80 to 1360, and contains a single base pair deletion at position 580. The 414 (NCoI) to 1360 (EcoRI) fragment of CH21.1C is spliced to CH8.2 (-40 to 414) to create the clone CH8.6 (-40 to 1360), with the single base deletion at position 580. This clone is deposited with the American Culture Collection and is Accession No. 68500. Also deposited is CH10.2M, a clone spanning positions 400 to 1360, but with a 17-base pair insertion at position 580.

4. Expression of the Chicken Growth Hormone Receptor in Tissue Culture

The full length chicken growth hormone receptor clone, pCH21.9F (pGEM-cGHR), is treated with restriction enzymes Xho I and Sac I to remove the fragment containing the chicken growth hormone receptor. This fragment is isolated and ligated into the mammalian expression vector pSVL (Pharmacia) which had previously been cut with the same restriction enzymes. The resulting plasmid, pSVL-cGHR, is introduced into mammalian cells.

CMT-3 cells, of monkey kidney origin, are grown in standard tissue culture medium (DMEM + 10% fetal bovine serum) at 37°C in 5% $CO_2$. The plasmid pSVL-cGHR is introduced into $10^5$ cells/10 cm plate using Lipofectin Reagent (BRL, Maryland) according to the manufacturer's instructions. After 48 hours incubation, the cells are washed twice in PBS and incubated for an additional 24 hours in a serum free medium, MCDB 201 (Sigma). At harvest the medium is removed and saved. The cells are scraped into PBS and washed twice with PBS. Cell associated growth hormone binding activity is measured by incubating the cells in PBS containing 0.5% bovine serum albumin, 10mM $MgCl_2$ with 40,000 cpm of $I^{125}$-bovine growth hormone (50-60 uCi/ug) and increasing amounts of unlabelled bovine growth hormone. Bound and free fractions are separated by centrifugation. Growth hormone binding activity in the medium is detected by incubating in 0.5% BSA and 10mM $MgCl_2$ with increasing amounts of $I^{125}$-bovine growth hormone. Bound and free fractions are separated by chromatography on a Sephacryl S-200 column as described (Ymer and Herington, Mol. Cell Endo 41:153-161, 1985; Baumann et al., J. Clin. Endo. Metab. 62: 1986; Herington et al., J. Clin. Invest. 77, 1986).

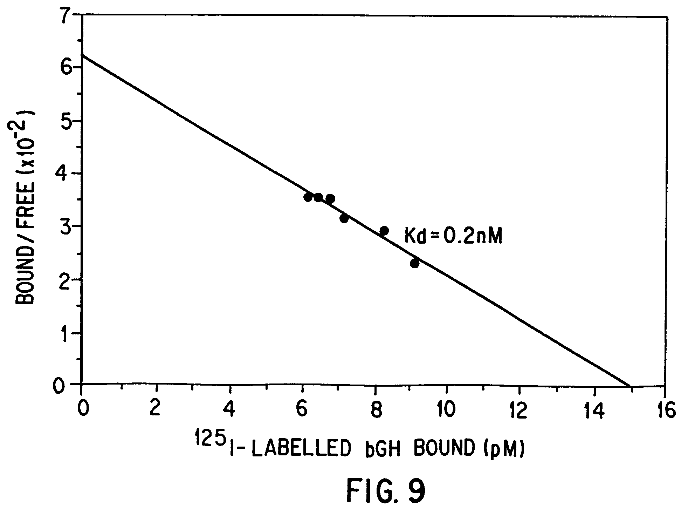

The above protocol is employed in COS-7 cells with the following modifications: the plasmid is introduced into 5 x$10^5$ cells/10 cm plate with lipofectin Opti-MEMI medium (Gibco). After 24 hours, incubation, cells are washed twice and incubated in serum free medium PFHM-II (Gibco). Results of GH binding to expressed receptor is presented in a Scatchard plot in Figure 9.

5. Introduction of the Chicken Growth Hormone Receptor into Chicken Embryos

The full-length chicken GH receptor is introduced into retroviral vectors based on the strain of avian reticuloendotheliosis virus called SNV (spleen necrosis virus) (Shimotohno and Temin, Cell 26:67-77, 1981). These vectors contain retroviral elements (including LTRs and encapsidation sequences) sufficient for transcription of RNA that can be packaged into infectious, replication incompetent virions containing sequences encoding the chicken GHR. This construct is introduced through DNA transfection into the canine cell line C3 which contains two defective helper proviruses, one that provides gag and pol RNA and one that provides env RNA. Packaging sequences (required for viral encapsidation) have been deleted from these proviruses (Watanabe and Temin, PNAS USA 79:5986-5990, 1983). Thus, no replication competent virus is produced and infectious, replication defective virus containing the chicken GHR gene is secreted by this cell line. Infectious supernatants are collected and used to infect chicken embryos.

The plasmid pPB101 (Bandyopadhyay and Temin, Mol. Cell. Biol., 4:743-748, 1984) contains the entire SNV genome. The fragment from the XmaIII site at position 0.975 kbp to the BglII site at position 6.7 kbp in SNV (containing the viral structural genes gag, pol and env) is removed from this plasmid, and is replaced by the chicken GHR cDNA. Thus, the initiation ATG codon for translation of the RNA encoded by this provirus is from the chicken GHR (O'Rear and Temin, PNAD USA, 79:1230-1234, 1982). The cis-acting functions needed for viral replication (the LTRs, the primer binding site, the encapsidation sequence and the polypurine track are encoded in the chicken GHR pro-virus, and the transacting components (gag, pol and env gene products) are provided by the recipient C3 cells. The resultant plasmid, pSNV-CGHR, is introduced into the C3 cell line by lipofection (Lipofectin, BRL, Maryland) in the presence of the dominant selectable marker enclded by the plasmid SV2-$gpt^r$ (Mulligan and Berg, PNAS USA, 78:2072-2076, 1981) which allows isolation of colonies in the presence of mycophenolic acid.

Clones of helper cells producing chicken GHR RNA are selected and used to produce infectious supernatants which are injected into unincubated chicken eggs, as described (Bosselman et al., 1989). The production of RNA encoding the exogenous chicken GHR is detected in Northern analysis of chicken tissues by the appearance of the uniquely sized mRNA produced by the retroviral vector.

6. Expression of a Potential Chicken Growth Hormone Binding Protein in Tissue Culture

The cDNA clone CH21.9A, which includes a frameshift relative to the expected chicken growth hormone receptor sequence (based on homology to mammalian growth hormone receptors) is digested with Xho I and Sac I, and cloned into the mammalian expression vector pSVL (see above) for expression in CMT-3 cells. An identical protocol is followed for expression of the construct except that the tissue culture supernatant is collected for analysis of growth hormone binding activity in the form of a secreted, soluble growth hormone binding protein. Growth hormone binding activity is detected by incubating this supernatant in 0.5% BSA and 10mM $MgCl_2$ with increasing amounts of $I^{125}$-bovine growth hormone. Bound and free fractions are separated by chromatography on a Sephacryl S-200 column as described (Ymer and Herington, supra; Baumann et al. supra; Herington et al., supra).

7. Northern Analysis of Chicken mRNA

Cytoplasmic RNA is prepared from liver tissue of broiler pullets and cockerels as described (Favaloro et al., Meth. Enzymol. 65:718, 1980) and polyA+ RNA was selected by chromatography on oligo dT cellulose (Maniatis et al., Molecular Cloning, 2nd Ed., 1989). For Northen analysis, 3μg polyA+ RNA is electrophoresized in a 1% agarose gel containing formaldehyde and transferred to nylon (Nytran, S&S) as described. The membranes are prehybrized and hybridized in Church buffer. The blots are washed in 0.2 x SSC and 0.1% at 65°C. Blots were exposed at -70°C to x-ray film with an intensifying screen. All probes are labelled with $\alpha$-$^{32}$P-dCTP by random priming. The probe is CH8.6, which would recognize both full length receptor mRNA and GH binding protein mRNA. Two species of mRNA were detected, at 4.5 kb and 0.9 kb. The large species is similar in size to GH receptor mRNA from other species, but the 0.9 kb species is smaller than other GH binding protein mRNAs.

The patterns of expression observed in liver through ontogeny show that very young birds express much less GH receptor mRNA than their older counterparts (Table 2).

TABLE 2

| Ontogeny of Growth Hormone Receptor mRNA Levels in Chickens[2] | | |
|---|---|---|
| | mRNA Levels (AU x $10^3$)[1] | |
| | 4.5 kb cGHR band | 0.90 kb band |
| > 1 year female | 7.01 | 1.19 |
| + 14 week femals | 7.38 | 2.75 |
| + 8 week female | 8.76 | 3.95 |
| + 6 week male | 4.21 | 2.67 |
| + 6 week female | 5.16 | 1.40 |
| + 5 week male | 3.15 | 1.00 |
| + 5 week female | 2.78 | 0.97 |
| + 4 week male | 1.01 | 0.68 |
| + 4 week female | 1.52 | 0.79 |
| + 3 week male | 1.20 | 0.64 |
| + 3 week female | 2.31 | 0.48 |
| + 2 week male | 3.95 | 1.69 |
| + 2 week female | 1.11 | 4.77 |
| + 1 week male | 0.21 | 5.96 |
| + 1 week female | 0.51 | 4.95 |
| + 4 day male | 1.55 | 3.76 |
| + 4 day female | 2.24 | 2.06 |
| + 1 day male | 0.47 | 1.52 |
| + 1 day female | 0.63 | 0.21 |

Notes: 1. mRNA levels are expressed as arbitrary densitometric units (AU).
2. Each point represents mRNA made from pooled frozen livers from 6 birds.
Broilers used were A.A. X A.A. crosses, raised under standard floor pen conditions.

8. Identification of small mRNA hybridizing to chicken GH receptor

Further inspection of the smaller RNA species identified in Example 7 reveals the actual size to be about 0.7-0.9 kb. The size of the message without the tail is approximately 0.5 kb.

To characterize the structure of this mRNA, a library is made from cDNA that is size selected on an agarose gel and shown by hybridization with the CH8.6 probe to correspond specifically to the small mRNA of interest.

To construct the library, mRNA that has been shown to contain a relatively high level of this 0.5 kb species (combined from one week old male and female broilers, see Example 7, Table 2) is used to produce cDNA. A cDNA library is constructed using modifications of the Superscript Lambda Cloning System (BRL, Life Technologies, Inc., Grand Island, NY). First strand synthesis of 20 ug poly(A)+ RNA, 10 µg each of 1 week old male and female liver, is primed by an oligo (dT) Not I primer-adapter which contains 15 dT residues and four restriction endonuclease sites (NotvI, Nru I, Xba I and Spe I) found infrequently in mammalian genomes. Second strand synthesis is catalyzed by E. coli DNA polymerase I in combination with E. coli RNase H and E. coli DNA ligase. The termini of cDNAs are blunted by T4 DNA polymerase. The reaction mixture is then deproteinized by organic extraction and precipitated with ethanol. To maximize ligation efficiency into the vector, a Sal I adapter is spliced to the blunt end of cDNA molecules by T4 DNA ligase. By using both Sal I and Not I adapters, directional cloning of the insert is obtained by introducing two different restriction endonuclease sites at the 5' and 3' ends, respectively (Table 3).

**TABLE 3**

**Sequences of Not I Primer-Adapter and Sal I Adapter for Construction of cDNA Library**

**Not I Primer-adapter**

**5'-pGACTAGTTCTAGATCGCGAGCGGCCGCCC(T)$_{15}$-3'**

**Sal I Adapter**

**5'-pTCGACCCACGCGTCCG-3'**

**3'-    GGGTGCGCAGGCp-5'**

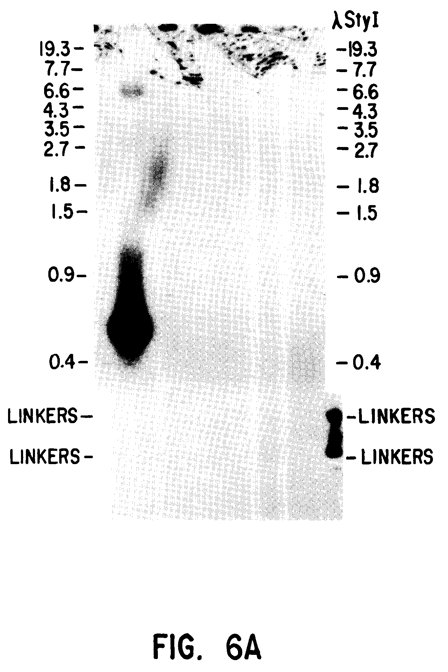

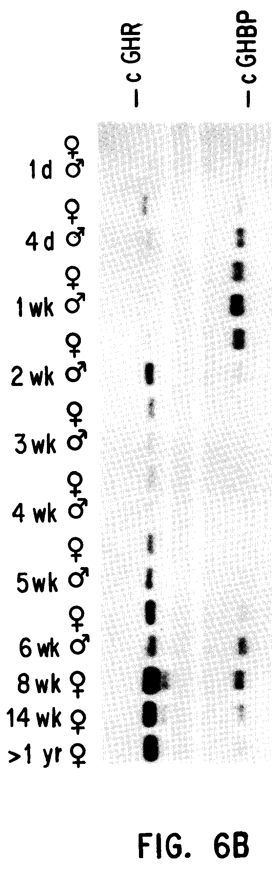

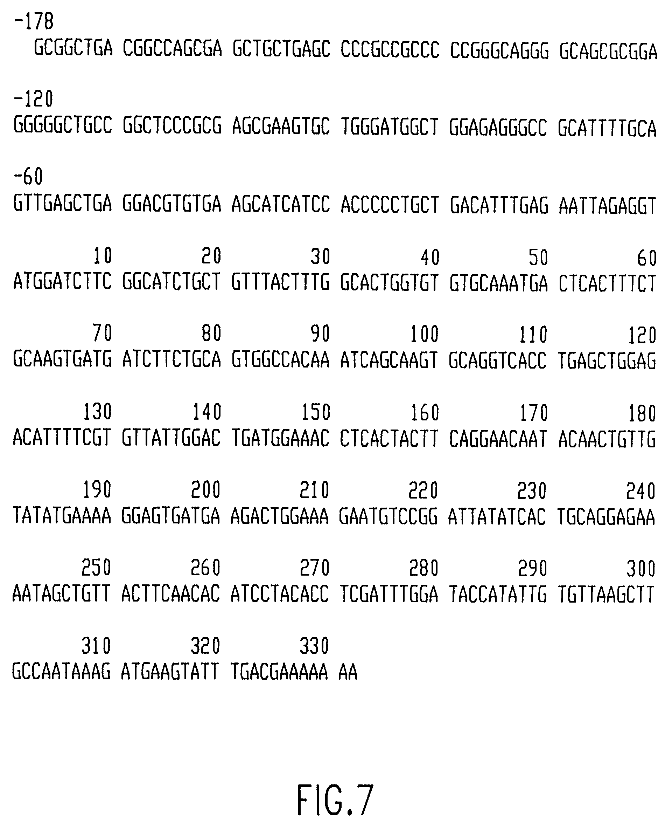

This ligation reaction is again deproteinized by organic extraction, precipitated with ethanol and then digested with the restriction endonuclease, Not I. An aliquot of this digestion mixture is size fractionated by electrophoresis in a 0.8% agarose gel and transferred to nylon (Nytran, Schleicher & Schuell, Keene, NJ). The membrane is prehybridized and hybridized in Church buffer. The blot is washed four tiems: first, at 25°C in 2x SSC, 0.2% SDS and three times at 65°C in 0.2x SSC, 0.2% SDS. The blot is exposed at -70°C to x-ray film with an intensifying screen. The probe is a gel purified 450 nucleotide EcoRI/NcoI fragment excised from avian GH receptor clone C23.6A and is labeled with $\alpha$-$^{32}$P-dCTP by random priming. This probe corresponds to nucleotide positions -40 to 414 of the full length chicken GH receptor. Three species of cDNA are detected, at approximately 4.5 kb, 4.0 kb and 0.5 kb (Figure 6A). All species correspond in size to those observed in Northern analysis (Figure 6B).

To isolate and characterize the 0.5 kb species, the remaining reaction mixture is size fractionated in a 0.8% garose gel containing formaldehyde as described in Example 7. The fraction of the cDNA library migrating at 0.5 kb is excised from the gel. cDNA is isolated by electroelution (Maniatis, et al., Molecular Cloning, 2nd Ed., 1989) and further purified on an Econo-Pac 10DG (BioRad, Richmond, CA) and ethanol precipitated. The cDNA is ligated to lambda gt23A Not I-Sal I arms (BRL, Life Technologies, Inc., Grand Island, NY) and packaged using Gigapack Gold (Stratagene, La Jolla, CA).

E. coli strain Y1090 is infected with the entire library (approximately $10^6$ clones), and plated on six 150 mm plates. Nitrocellulose plaque lifts are taken from each plate. The filters are probed with the 450 nucleotide EcoRI/NcoI fragment described above. Filters are prehybridized and hybridized in Church buffer. Filters are washed four times: first, at 25°C in 2x SSC, 0.2% SDS and then three times at 65°C in 0.2x SSC, 0.2% SDS. Filters are exposed to film with intensifying screens. Seven positives are chosen for secondary and tertiary screening. Six of the seven clones are retested through the screening procedure. Inserts from these six phage are excised by cleavage with Not I and Sal I, gel purified and subcloned into the plasmid pSPORT (BRL, Life Technologies, Inc., Grand Island, NY).

9. Sequence Analysis

Sequencing is performed using the Sequence System (United States Biochemical, Cleveland, OH) protocol for double stranded DNA. All sequencing reactions used $^{35}$S-dATP as a radioactive label and are run on 6% polyacrylamide/urea gels. Each insert subcloned in the plasmid is sequenced from the polyacrylamide/urea gels. Each insert subcloned in the plasmid is sequenced from each end using primers that annealed outside the pSPORT polylinker (e.g. the T7 and SP6 primers). In addition, synthetic oligonucleotide primers are used. The nucleotide sequence and annealing sites of the synthetic oligonucleotides are listed in Table 4.

**TABLE 4**

**Synthetic Oligonucleotides and Annealing Sites**

| | | |
|---|---|---|
| CH 12 | 5'-dTGGATCTTCGGCATCTGCTG-3' | 178-200 |
| CH 19 | 5'-dTTCATATACAACAGTTGTAT-3' | 346-366 |

Sequence analysis of the cDNAs reveals that four of the six clones contain the first 325 nucleotides of the chicken GH receptor coding region corresponding to mammalian exons 2, 4 and 5 (there is no exon 3 in birds). All clones possess varying amounts of the 5' untranslated region; clone CH 500.2, CH 500.5, CH 500.6 and CH 500.7 contain 178, 44, 117 and 117 nucleotides of the 5' untranslated region, respectively. Clone CH 500.2 (Figure 7) is likely to represent the full length version of this RNA species, and has been deposited with the American Type Culture Collection (Accession Number 68621). The remaining two clones fail to show any similarity to the known avian GH receptor and are not analyzed further.

Each of the cDNA clones reveal an identical structure at their 3' end, where the apparent addition of the poly(A) tail occurs after nucleotide position 325. This position is located 17 bases after the sequence AATAAA (position 304-309), which is the concensus poly(A) addition signal in eukaryotes (Wickens, M., TIBS 15:277-281, 1990). Poly(A) addition is predicted to occur 10 to 30 nucleotides after the AATAAA concensus sequence. In addition, a diffuse GT-rich region ranging from 0 to 10 nucleotides 3' of the poly(A) addition site is the other required element in mammalian and other higher eukaryotic poly(A) addition signals (Proudfoot, N., Cell 64:671-674, 1991). The sequence following the putative cleavage site in the chicken GH receptor is AAAAGTGTTTCAGTGTTG, a motif that fulfills the predicted requirement for the downstream poly(A) addition element. The actual cleavage site may occur after any one of the 4 A's in positions 326-329 without changing the final structure of this message. Thus, the short (0.5 kb) chicken GH receptor RNA species is highly likely to be the result of cleavage and polyadenylation of the primary GH receptor transcript. It should be noted that this class of transcripts has no stop codon, and is thus unlikely to be efficiently translated. If translation did occur, multiple lysine residues would result from reading of the poly(A) tail of the truncated message.

10. Northern Analysis of the 0.5 kb Transcript in Domestic and Wild Avian Species

Cytoplasmic and poly(A)$^+$ RNA are prepared from livers of domestic broiler breeders (AA x AA) and quail as previously described in Example 7. For Northern analysis, 3 ug of poly(A)$^+$ RNA are electrophoresed in a 0.8% agarose gel containing formaldehyde and analyzed as described above. The probe is a 306 nucleotide HindIII/EcoRI fragment excised from pSPORT CH 500.5, containing 44 bases of the 5' untranslated region and exons 2, 4 and 5; the fragment is labeled with $\alpha$-$^{32}$P-dCTP by random priming and should recognize both full length receptor and 0.5 kb mRNA. In both chicken and quail, three abundant species of mRNA (4.5 kb, 4.0 kb and 0.5 kb) are detected (Figure 8). Thus, it is likely that a similar mechanism exists in quail for the production of the 0.5 kb transcript. In addition to the three major species of mRNA in common with chicken, quail contain an additional abundant mRNA of approximately 1.2 kb which appears to be expressed only at low levels in chicken. This species may encode a genuine GHBP, based upon its similarity in size to the GHBP mRNA found in rat.

11. Mutagenesis of the Chicken GH Receptor to Eliminate Alternative Polyadenylation of mRNA

The poly(A) addition consensus sequence, AATAAA, found at position 304-309 can be altered through site directed mutagenesis to AACAAG without affecting the translated amino acid sequence of the GH receptor (the amino acids Asn-Lys are encoded by either of these nucleotide sequences). This altered sequence can be used to construct expression vectors for use in transgenic chickens or in vitro tissue culture systems. Such vectors should encode mRNAs that are not subject to alternative polyadenylation, because each of the two separate nucleotide changes would be expected to reduce the utilization of the site by >95% (Wickens, 1990, supra). Comparisons between cell lines expressing wild type and mutated versions of the cloned chicken GH receptor cDNA will verify the abolition of alternative polyadenylation when these changes are made.

12. Expression of the 0.5 kb Transcript in Tissue Culture

COS-7 cells, of monkey kidney origin are grown in standard tissue culture medium (DMEN + 10% fetal bovine serum) at 37°C in 5% $CO_2$. Cells are transfected, as previously described, with plasmid pSVL-cGHR containing the consensus sequence for the poly(A) addition in exon 5 or one in which this site has been altered. Cytoplasmic RNA is prepared from $10^7$ cells as described (Favaloro et al. Meth. Enzymol. 65:716, 1980) and poly(A) RNA was selected by chromatography with oligo(dT) cellulose (Maniatis, et al., Molecular Cloning, 2nd Ed., 1989). Northern analysis is performed as described. The membrane is prehybridized and hybridized in Church buffer. The blot is washed four times: first, at 25°C in 2x SSC, 0.2% SDS and the three times at 65°C in 0.2x SSC, 0.2% SDS. The blot is exposed at -70°C to x-ray film with an intensifying screen. The probe is the 306 nucleotide HindIII/EcoRI fragment from clone CH 500.5, and is labeled with $\alpha$-$^{32}$P-dCTP by random priming. Results from Northern analysis show that 2 species of mRNA corresponding to the full length receptor and 0.5 kb transcript are processed by these cells (Figure 8).

13. S1 Nuclease Analysis of 0.5 kb Transcript

S1 nuclease analysis is used to map the 3' end of the 0.5 kb transcript (Maniatis, et al., Molecular Cloning, 2nd Ed., 1989). An oligonucleotide consisting of 61 nucleotides complementary to bases 462 to 513 of CH 500.2 is designed (Table 5, regions 1 and 2). The 5' end of this oligonucleotide possess a string of 10 dT (Table 5, region 1) while the 3' end had 10 additional bases which did not complement the sequence (Table 5, region 3). In this manner the full length probe could be distinguished from the protected fragment created during hybridization and subsequent digestion. The 5' end of this oligonucleotide is labeled with $\gamma$-$^{32}$P-dATP by T4 polynucleotide kinase and hybridized overnight to chicken mRNA at 30°C in 80% formamide hybridization buffer. S1 nuclease is added to digest any single stranded regions failing to hybridize to appropriate mRNA. This mixture is ethanol precipitated, resuspended in formamide and run on a 10% polyacrylamide/urea gel. The protected fragment should be 51 nucleotides in size and correspond to regions 462 through 513 of CH 500.2.

**TABLE 5**

**Synthetic Oligonucleotide Used to Map**

**3' End of 0.5 kb Transcript in Chickens**

**Region 1**

**5'-dTTTTTTTTTT**

**Region 2**

**CGTCAAATACTTCATCTTTATTGGCAAGCTTAACACAATAT**

**Region 3**

**CATAGGTT-3'**

14. Analysis of Clone Having 17 bp Insert

The chicken GH cDNA clone, 10.2M, includes an insertion of 17 bp with respect to the normal, full length chicken GH receptor cDNA. To show that this variation is the result of alternative splicing of GH receptor mRNA, chicken genomic DNA is analyzed in the region corresponding to the intron/exon junction predicted to be found at the point of this insertion. A genomic $\gamma$-phage library made from male broiler chicken DNA (Clontech) is separately probed with radiolabeled fragments corresponding to exons 6 and 7 of the human GH receptor (Godowski et al., PNAS USA 86:8083-8087, 1989). A $\gamma$clone with an insert size of approximately 10 kb, called CH63.1, is isolated and shown to hybridize with each of these two probes, suggesting that this clone encompasses most or all of exons 6 and 7 and the entire intervening sequence between them (IVS-6). Oligonucleotide sequencing primers in each of exons 6 and 7 are used to initiate sequencing reactions directed across the exon/intron junctions into IVS-6. The results of this analysis showed that the 17 base pairs are found to be inserted in the GH receptor cDNA are found within IVS-6, immediately 5' to the junction with exon 7 (Table 6). Thus, it is likely that, in the chicken, an alternative splice acceptor site leads to the production of mRNA that encodes a truncated, secreted GH receptor molecule, similar in structure to the GH binding protein found in mammals.

**TABLE 6**

**<u>Exon 6 - IVA 6 Junction</u>**

**AAA GAA GTT AAT GAG**
**Lys Glu Val Asn Glu**

**ACAAAATGGAAGGAGGTATGGAGGAAATATTTATTATGCAGTAGTCATGGTGCAG**

**<u>IVS 6 - Exon 7 Junction</u>**

**TAGAATTGCAGGTACAACTCACACAAATGTATCACAG CATTTCTGTCTTTGCAG**
**17 bp insert**

**TTA GAA CCC AGG CTC**
**Leu Glu Pro Arg Leu**

15. <u>Screen for Compounds Inhibiting Utilization of Poly(A) Site</u>

To find compounds or biological factors that can induce GH receptor levels in young chickens or other poultry through regulation of alternative poly(A) site selection, <u>in vitro</u> expression systems are used as screening tools. A reporter gene such as luciferase that can be detected enzymatically is fused to the 5' end of the chicken GH receptor. This fusion gene, driven by a powerful constitutive promoter, has its own poly(A) site, but the alternative poly(A) site in the GH receptor coding region will compete for cleavage and polyadenylation with the luciferase poly(A) site. This construct is transfected into an avian cell line that shows preferential utilization of the upstream poly(A) site. Treatment of this cell line in culture with factors or compounds is followed by enzymatic assays for luciferase. Increases in luciferase activity will indicate altered regulation of the poly(A) site utilization, such that less truncated message is formed, and more full length mRNA is produced.

**<u>DEPOSIT OF BIOLOGICAL MATERIALS</u>**

The following plasmids have been deposited with the American Type Culture Collection, 12301 Parklawn Drive, Rockville, Maryland, and have been given accession numbers as follows (all deposits in <u>E. coli</u> strain DH5α):

| Plasmid | Accession No. |
|---|---|
| pCH10.2M | 68498 |
| pCH21.9F | 68499 |
| pCH8.6 | 68500 |
| pSPORTch 500.2 | 68621 |

Sequence ID No.: 1

Sequence Type: Nucleic Acid and Amino Acid

Sequence Length: 549 Base Pairs
183 Amino Acid Residues

Strandedness: Single

Topology: Linear

Original Source Organism: Chicken

Properties: Chicken Growth Hormone Receptor Gene with 17 Base Pair Insertion, and Corresponding Protein

```
ATG GAT CTT CGG CAT CTG CTG TTT ACT TTG GCA CTG      36
Met Asp Leu Arg His Leu Leu Phe Thr Leu Ala Leu
1               5                   10

GTG TGT GCA AAT GAC TCA CTT TCT GCA AGT GAT GAT      72
Val Cys Ala Asn Asp Ser Leu Ser Ala Ser Asp Asp
        15                  20

CTT CTG CAG TGG CCA CAA ATC AGC AAG TGC AGG TCA     108
Leu Leu Gln Trp Pro Gln Ile Ser Lys Cys Arg Ser
25                  30                  35

CCT GAG CTG GAG ACA TTT TCG TGT TAT TGG ACT GAT     144
Pro Glu Leu Glu Thr Phe Ser Cys Try Trp Thr Asp
            40                  45

GGA AAC CTC ACT ACT TCA GGA ACA ATA CAA CTG TTG     180
Gly Asn Leu Thr Thr Ser Gly Thr Ile Gln Leu Leu
    50                  55                  60
```

```
TAT ATG AAA AGG AGT GAT GAA GAC TGG AAA GAA TGT      216
Tyr Met Lys Arg Ser Asp Glu Asp Trp Lys Glu Cys
                65                      70

CCG GAT TAT ATC ACT GCA GGA GAA AAT AGC TGT TAC      252
Pro Asp Tyr Ile Thr Ala Gly Glu Asn Ser Cys Tyr
        75                  80

TTC AAC ACA TCC TAC ACC TCG ATT TGG ATA CCA TAT      288
Phe Asn Thr Ser Tyr Thr Ser Ile Trp Ile Pro Tyr
85                  90                  95

TGT GTT AGG CTT GCC AAT AAA GAT GAA GTA TTT GAC      324
Cys Val Lys Leu Ala Asn Lys Asp Glu Val Phe Asp
            100                 105

GAA AAG TGT TTC AGT GTT GAT GAA ATA GTA CTA CCT      360
Glu Lys Cys Phe Ser Val Asp Glu Ile Val Leu Pro
    110                 115                 120

GAT CCC CCT GTG CAC CTT AAC TGG ACT CTG CTA AAT      396
Asp Pro Pro Val His Leu Asn Trp Thr Leu Leu Asn
                125                 130

ACT AGT CAA ACT GGG ATC CAT GGG GAT ATT CAA GTA      432
Thr Ser Gln Thr Gly Ile His Gly Asp Ile Gln Val
        135                 140

CGA TGG GAT CCA CCA CCA ACA GCA GAT GTT CAG AAA      468
Arg Trp Asp Pro Pro Pro Thr Ala Asp Val Gln Lys
145                 150                 155

GGA TGG ATT ACT CTG GAG TAT GAA TTG CAG TAC AAA      504
Gly Trp Ile Thr Leu Gly Tyr Glu Leu Gln Tyr Lys
            160                 165
```

GAA GTT AAT GAG ACA AAA TGG AAG GAG CAT TTC TGT 540
Glu Val Asn Glu Thr Lys Trp Lys Glu His Phe Cys
170 175 180

CTT TGC AGT 549
Leu Cys Ser
183

Sequence ID No.: 2

Sequence Type: Nucleic Acid and Amino Acid

Sequence Length: 723 Base Pairs
241 Amino Acid Residues

Strandedness: Single

Topology: Linear

Original Source Organism: Chicken

Properties: Chicken Growth Hormone
Binding Protein Gene and Protein

```
ATG GAT CTT CGG CAT CTG CTG TTT ACT TTG GCA CTG      36
Met Asp Leu Arg His Leu Leu Phe Thr Leu Ala Leu
1               5                   10

GTG TGT GCA AAT GAC TCA CTT TCT GCA AGT GAT GAT      72
Val Cys Ala Asn Asp Ser Leu Ser Ala Ser Asp Asp
        15                  20

CTT CTG CAG TGG CCA CAA ATC AGC AAG TGC AGG TCA     108
Leu Leu Gln Trp Pro Gln Ile Ser Lys Cys Arg Ser
25                  30                  35

CCT GAG CTG GAG ACA TTT TCG TGT TAT TGG ACT GAT     144
Pro Glu Leu Glu Thr Phe Ser Cys Tyr Trp Thr Asp
            40                  45

GGA AAC CTC ACT ACT TCA GGA ACA ATA CAA CTG TTG     180
Gly Asn Leu Thr Thr Ser Gly Thr Ile Gln Leu Leu
    50                  55                  60
```

| | | | | | | | | | | | | |
|---|---|---|---|---|---|---|---|---|---|---|---|---|
| TAT | ATG | AAA | AGG | AGT | GAT | GAA | GAC | TGG | AAA | GAA | TGT | 216 |
| Tyr | Met | Lys | Arg | Ser | Asp | Glu | Asp | Trp | Lys | Glu | Cys | |
| | | | | 65 | | | | | 70 | | | |
| CCG | GAT | TAT | ATC | ACT | GCA | GGA | GAA | AAT | AGC | TGT | TAC | 252 |
| Pro | Asp | Tyr | Ile | Thr | Ala | Gly | Glu | Asn | Ser | Cys | Tyr | |
| | | 75 | | | | | 80 | | | | | |
| TTC | AAC | ACA | TCC | TAC | ACC | TCG | ATT | TGG | ATA | CCA | TAT | 288 |
| Phe | Asn | Thr | Ser | Tyr | Thr | Ser | Ile | Trp | Ile | Pro | Tyr | |
| 85 | | | | | 90 | | | | | 95 | | |
| TGT | GTT | AAG | CTT | GCC | AAT | AAA | GAT | GAA | GTA | TTT | GAC | 324 |
| Cys | Val | Lys | Leu | Ala | Asn | Lys | Asp | Glu | Val | Phe | Asp | |
| | | | 100 | | | | | 105 | | | | |
| GAA | AAG | TGT | TTC | AGT | GTT | GAT | GAA | ATA | GTA | CTA | CCT | 360 |
| Glu | Lys | Cys | Phe | Ser | Val | Asp | Glu | Ile | Val | Leu | Pro | |
| | 110 | | | | | 115 | | | | | 120 | |
| GAT | CCC | CCT | GTG | CAC | CTT | AAC | TGG | ACT | CTG | CTA | AAT | 396 |
| Asp | Pro | Pro | Val | His | Leu | Asn | Trp | Thr | Leu | Leu | Asn | |
| | | | | 125 | | | | | 130 | | | |
| ACT | AGT | CAA | ACT | GGG | ATC | CAT | GGG | GAT | ATT | CAA | GTA | 432 |
| Thr | Ser | Gln | Thr | Gly | Ile | His | Gly | Asp | Ile | Gln | Val | |
| | | 135 | | | | | 140 | | | | | |
| CGA | TGG | GAT | CCA | CCA | CCA | ACA | GCA | GAT | GTT | CAG | AAA | 468 |
| Arg | Trp | Asp | Pro | Pro | Pro | Thr | Ala | Asp | Val | Gln | Lys | |
| 145 | | | | | 150 | | | | | 155 | | |
| GGA | TGG | ATT | ACT | CTG | GAG | TAT | GAA | TTG | CAG | TAC | AAA | 504 |
| Gly | Trp | Ile | Thr | Leu | Glu | Tyr | Glu | Leu | Gln | Tyr | Lys | |
| | | | 160 | | | | | 165 | | | | |

```
GAA GTT AAT GAG ACA AAA TGG AAG GAG TTA GAA CCC      540
Glu Val Asn Glu Thr Lys Trp Lys Glu Leu Glu Pro
    170                 175                 180

AGG CTC TCA ACA GTG GTT CCA CTG TAT TCT CTG AAG      576
Arg Leu Ser Thr Val Val Pro Leu Tyr Ser Leu Lys
                185                 190

ATG GAA GAG ATT ATG AGA TCC GAG TCC GAT CAA GAC      612
Met Glu Glu Ile Met Arg Ser Glu Ser Asp Gln Asp
        195                 200

AAC GTA CCT CCG AAA AGT TTG GGG AGT TCA GTG AAA      648
Asn Val Pro Pro Lys Ser Leu Gly Ser Ser Val Lys
205                 210                 215

TCC TCT ATG TTT CCT TTA CTC AAG CAG GCA TTG AAT      684
Ser Ser Met Phe Pro Leu Leu Lys Gln Ala Leu Asn
            220                 225

TTG TTC ATT GTG CTG AAG AAA TCG AGT TTC CCT GGT      720
Leu Phe Ile Val Leu Lys Lys Ser Ser Phe Pro Gly
    230                 235                 240

TCT                                                  723
Ser
241
```

Sequence ID No.: 3

Sequence Type: Nucleic Acid and Amino Acid

Sequence Length: 1824 Base Pairs
608 Amino Acid Residues

Strandedness: Single

Topology: Linear

Original Source Organism: Chicken

Properties: Chicken Growth Hormone
Receptor Gene and Protein

```
ATG GAT CTT CGG CAT CTG CTG TTT ACT TTG GCA CTG      36
Met Asp Leu Arg His Leu Leu Phe Thr Leu Ala Leu
1               5                   10

GTG TGT GCA AAT GAC TCA CTT TCT GCA AGT GAT GAT      72
Val Cys Ala Asn Asp Ser Leu Ser Ala Ser Asp Asp
        15                  20

CTT CTG CAG TGG CCA CAA ATC AGC AAG TGC AGG TCA     108
Leu Leu Gln Trp Pro Gln Ile Ser Lys Cys Arg Ser
25                  30                  35

CCT GAG CTG GAG ACA TTT TCG TGT TAT TGG ACT GAT     144
Pro Glu Leu Glu Thr Phe Ser Cys Tyr Trp Thr Asp
            40                  45

GGA AAC CTC ACT ACT TCA GGA ACA ATA CAA CTG TTG     180
Gly Asn Leu Thr Thr Ser Gly Thr Ile Gln Leu Leu
    50                  55                  60
```

TAT ATG AAA AGG AGT GAT GAA GAC TGG AAA GAA TGT 216
Tyr Met Lys Arg Ser Asp Glu Asp Trp Lys Glu Cys
65 70

CCG GAT TAT ATC ACT GCA GGA GAA AAT AGC TGT TAC 252
Pro Asp Tyr Ile Thr Ala Gly Glu Asn Ser Cys Tyr
75 80

TTC AAC ACA TCC TAC ACC TCG ATT TGG ATA CCA TAT 288
Phe Asn Thr Ser Tyr Thr Ser Ile Trp Ile Pro Tyr
85 90 95

TGT GTT AAG CTT GCC AAT AAA GAT GAA GTA TTT GAC 324
Cys Val Lys Leu Ala Asn Lys Asp Glu Val Phe Asp
100 105

GAA AAG TGT TTC AGT GTT GAT GAA ATA GTA CTA CCT 360
Glu Lys Cys Phe Ser Val Asp Glu Ile Val Leu Pro
110 115 120

GAT CCC CCT GTG CAC CTT AAC TGG ACT CTG CTA AAT 396
Asp Pro Pro Val His Leu Asn Trp Thr Leu Leu Asn
125 130

ACT AGT CAA ACT GGG ATC CAT GGG GAT ATT CAA GTA 432
Thr Ser Gln Thr Gly Ile His Gly Asp Ile Gln Val
135 140

CGA TGG GAT CCA CCA CCA ACA GCA GAT GTT CAG AAA 468
Arg Trp Asp Pro Pro Pro Thr Ala Asp Val Gln Lys
145 150 155

GGA TGG ATT ACT CTG GAG TAT GAA TTG CAG TAC AAA 504
Gly Trp Ile Thr Leu Glu Tyr Glu Leu Gln Tyr Lys
160 165

```
GAA GTT AAT GAG ACA AAA TGG AAG GAG TTA GAA CCC      540
Glu Val Asn Glu Thr Lys Trp Lys Glu Leu Glu Pro
    170                 175                 180

AGG CTC TCA ACA GTG GTT CCA CTG TAT TCT CTG AAG      576
Arg Leu Ser Thr Val Val Pro Leu Tyr Ser Leu Lys
                185                 190

ATG GGA AGA GAT TAT GAG ATC CGA GTC CGA TCA AGA      612
Met Gly Arg Asp Tyr Glu Ile Arg Val Arg Ser Arg
        195                 200

CAA CGT ACC TCC GAA AAG TTT GGG GAG TTC AGT GAA      648
Gln Arg Thr Ser Glu Lys Phe Gly Glu Phe Ser Glu
205                 210                 215

ATC CTC TAT GTT TCC TTT ACT CAA GCA GGC ATT GAA      684
Ile Leu Tyr Val Ser Phe Thr Gln Ala Gly Ile Glu
            220                 225

TTT GTT CAT TGT GCT GAA GAA ATC GAG TTT CCC TGG      720
Phe Val His Cys Ala Glu Glu Ile Glu Phe Pro Trp
    230                 235                 240

TTC TTA GTT GTT GTC TTC GGA GTG TGT GGG CTG GCC      756
Phe Leu Val Val Val Phe Gly Val Cys Gly Leu Ala
                245                 250

GTA ACA GCG ATC TTA ATC CTG TTG TCT AAA CAG CCA      792
Val Thr Ala Ile Leu Ile Leu Leu Ser Lys Gln Pro
        255                 260

AGG TTA AAA ATG CTG ATT TTT CCT CCT GTG CCA GTT      828
Arg Leu Lys Met Leu Ile Phe Pro Pro Val Pro Val
265                 270                 275
```

```
CCA AAG ATT AAA GGG ATT GAC CCA GAT CTC TTA AAG      854
Pro Lys Ile Lys Gly Ile Asp Pro Asp Leu Leu Lys
                280                 285

AAA GGA AAG CTA GAT GAA GTG AAC TCC ATC TTA GCC      900
Lys Gly Lys Leu Asp Glu Val Asn Ser Ile Leu Ala
    290                 295                 300

AGC CAT GAC AAC TAC AAG ACA CAG CTA TAC AAT GAT      936
Ser His Asp Asn Tyr Lys Thr Gln Leu Tyr Asn Asp
                    305                 310

GAC TTG TGG GTT GAG TTT ATT GAA TTG GAC ATA GAT      972
Asp Leu Trp Val Glu Phe Ile Glu Leu Asp Ile Asp
        315                 320

GAC TCC GAT GAA AAG AAC AGA GTC TCA GAT ACT GAC     1008
Asp Ser Asp Glu Lys Asn Arg Val Ser Asp Thr Asp
325                 330                 335

AGG CTC CTG AGT GAC GAT CAT CTG AAG TCA CAC AGT     1044
Arg Leu Leu Ser Asp Asp His Leu Lys Ser His Ser
                340                 345

TGC TTG GGA GCC AAG GAT GAT GAT TCT GGA CGT GCC     1080
Cys Leu Gly Ala Lys Asp Asp Asp Ser Gly Arg Ala
    350                 355                 360

AGT TGT TAT GAA CCA GAT ATT CCA GAG ACA GAC TTC     1116
Ser Cys Tyr Glu Pro Asp Ile Pro Glu Thr Asp Phe
                    365                 370

AGT GCA AGC GAC ACA TGT GAT GCC ATC TCT GAT ATT     1152
Ser Ala Ser Asp Thr Cys Asp Ala Ile Ser Asp Ile
        375                 380
```

GAT CAG TTC AAG AAG GTA ACT GAA AAA GAA GAG GAT 1188
Asp Gln Phe Lys Lys Val Thr Glu Lys Glu Glu Asp
385 390 395

CTC TTG TGC CTT CAT AGG AAA GAT GAT GTT GAG GCA 1224
Leu Leu Cys Leu His Arg Lys Asp Asp Val Glu Ala
400 405

CTT CAA AGT CTT GCC AAC ACA GAT ACC CAA CAG CCG 1260
Leu Gln Ser Leu Ala Asn Thr Asp Thr Gln Gln Pro
410 415 420

CAT ACT AGT ACT CAG TCT GAA AGC AGA GAG TCA TGG 1296
His Thr Ser Thr Gln Ser Glu Ser Arg Glu Ser Trp
425 430

CCA CCT TTT GCA GAC AGC ACT GAC TCA GCT AAT CCA 1332
Pro Pro Phe Ala Asp Ser Thr Asp Ser Ala Asn Pro
435 440

TCA GTC CAA ACT CAG CTA AGT AAC CAG AAT TCC CTG 1368
Ser Val Gln Thr Gln Leu Ser Asn Gln Asn Ser Leu
445 450 455

ACA AAC ACT GAC TTC TAT GCT CAA GTG AGT GAT ATT 1404
Thr Asn Thr Asp Phe Tyr Ala Gln Val Ser Asp Ile
460 465

ACT CCT GCT GGA AGT GTT GTA CTT TCT CCA GGG CAG 1440
Thr Pro Ala Gly Ser Val Val Leu Ser Pro Gly Gln
470 475 480

AAA TCC AAG GTG GGA AGA GCA CAG TGT GAA AGC TGC 1476
Lys Ser Lys Val Gly Arg Ala Gln Cys Glu Ser Cys
485 490

ACA GAA CAA AAC TTC ACC ATG GAC AAT GCC TAT TTC 1512
Thr Glu Gln Asn Phe Thr Met Asp Asn Ala Tyr Phe
495 500

TGT GAG GCA GAT GTG AAA AAA TGT ATT GCT GTG ATT 1548
Cys Glu Ala Asp Val Lys Lys Cys Ile Ala Val Ile
505 510 515

TCA CAG GAA GAG AAT GAG CCG CGT GTT CAG GAG CAA 1584
Ser Gln Glu Glu Asn Glu Pro Arg Val Gln Glu Gln
520 525

AGC TGT AAC GAG GAC ACT TAC TTC ACC ACA GAA AGC 1620
Ser Cys Asn Glu Asp Thr Tyr Phe Thr Thr Glu Ser
530 535 540

CTT ACC ACT ACC GGT ATC AAT CTT GGA GCT TCA ATG 1656
Leu Thr Thr Thr Gly Ile Asn Leu Gly Ala Ser Met
545 550

GCA GAA ACC CCA AGT ATG GAA ATG CCT GTC CCA GAC 1692
Ala Glu Thr Pro Ser Met Glu Met Pro Val Pro Asp
555 560

TAC ACT TCT ATT CAT ATT GTT CAC TCT CCA CAA GGC 1728
Tyr Thr Ser Ile His Ile Val His Ser Pro Gln Gly
565 570 575

CTT GTG CTC AAT GCA ACT GCA CTG CCT GTG CCA GAG 1764
Leu Val Leu Asn Ala Thr Ala Leu Pro Val Pro Glu
580 585

AAA GAA TTT AAC ATG TCT TGT GGC TAT GTG AGC ACA 1800
Lys Glu Phe Asn Met Ser Cys Gly Tyr Val Ser Thr
590 595 600

GAC CAG CTG AAC AAA ATC ATG CCG 1824
Asp Gln Leu Asn Lys Ile Met Pro
605 608

Sequence ID No.: 4

Sequence Type: Nucleic Acid

Sequence Length: 510 Base Pairs

Strandedness: Single

Topology: Linear

Original Source Organism: Chicken

Properties: Cleavage and Polyadenylation Product of Chicken Growth Hormone Receptor Gene

```
  GCGGCTGA CGGCCAGCGA GCTGCTGAGC CCCGCCGCCC          38

CCGGGCAGGG GCAGCGCGGA GGGGGCTGCC GGCTCCCGCG          78

AGCGAAGTGC TGGGATGGCT GGAGAGGGCC GCATTTTGCA         118

GTTGAGCTGA GGACGTGTGA AGCATCATCC ACCCCCTGCT         158

GACATTTGAG AATTAGAGGT                               178

ATG GAT CTT CGG CAT CTG CTG TTT ACT TTG GCA CTG     214

GTG TGT GCA AAT GAC TCA CTT TCT GCA AGT GAT GAT     250

CTT CTG CAG TGG CCA CAA ATC AGC AAG TGC AGG TCA     286

CCT GAG CTG GAG ACA TTT TCG TGT TAT TGG ACT GAT     322

GGA AAC CTC ACT ACT TCA GGA ACA ATA CAA CTG TTG     358
```

```
TAT ATG AAA AGG AGT GAT GAA GAC TGG AAA GAA TGT     394

CCG GAT TAT ATC ACT GCA GGA GAA AAT AGC TGT TAC     430

TTC AAC ACA TCC TAC ACC TCG ATT TGG ATA CCA TAT     466

TGT GTT AAG CTT GCC AAT AAA GAT GAA GTA TTT GAC     502

GAA AAA AA                                          510
```

## Claims

1. An isolated nucleic acid sequence encoding an avian growth hormone receptor protein.

2. The sequence of Claim 1 which encodes a chicken growth hormone receptor protein.

3. The sequence of Claim 1 which contains no polyadenylation site in the coding region.

4. The sequence of Claim 1 which is depicted in Figure 3, or fragments thereof encoding a biologically active peptide.

5. The sequence of Claim 3 in which the nucleotides at position 304-309 are replaced by AACAAG, AATAAG, or AACAAA.

6. The sequence of Claim 1 which is hybridizable with the sequence of Figure 3 under standard high stringency conditions.

7. An isolated avian growth hormone receptor protein.

8. The protein of Claim 1 which is a chicken growth hormone receptor protein.

9. The protein of Claim 7 which has the sequence depicted in Figure 1 or biologically active fragments thereof.

10. An isolated nucleic acid sequence encoding an avian growth hormone binding protein.

11. An isolated avian soluble growth hormone binding protein.

12. A method for producing an avian growth hormone receptor or growth hormone binding protein which comprises transforming a host cell with the sequence of Claim 1 or Claim 10 and culturing the host cell under conditions which permit expression of the protein.

13. The method of Claim 12 in which transformation is achieved by a virus or a plasmid.

14. An expression vector comprising the sequence of Claim 1, or Claim 3 or Claim 10.

15. A host cell comprising the sequence of Claim 1, or Claim 3 or Claim 10, or the expression vector of Claim 14.

**16.** A transgenic bird which is capable of expressing a growth hormone receptor protein at about eight weeks of age or less.

**17.** A pharmaceutical composition comprising an effective amount of an avian growth hormone receptor or an avian growth hormone binding protein, in combination with a physiologically acceptable carrier.

**18.** A method for modulating the activity of avian growth hormone in vivo which comprises administering to an animal in need of such treatment an effective amount of the protein of Claim 10.

**19.** A method for enhancing growth in birds comprising administering to a bird expressing a growth hormone receptor at an age of eight weeks or less an effective amount of an avian growth hormone.

**20.** A method for enhancing growth in birds comprising administering to a bird an effective amount of a compound which alters the normal pattern of utilization of polyadenylation site in the growth hormone receptor sequence.

```
                                    30                                    60
ATG GAT CTT CGG CAT CTG CTG TTT ACT TTG GCA CTG GTG TGT GCA AAT GAC TCA CTT TCT
Met Asp Leu Arg His Leu Leu Phe Thr Leu Ala Leu Val Cys Ala Asn Asp Ser Leu Ser

                                    90                                   120
GCA AGT GAT GAT CTT CTG CAG TGG CCA CAA ATC AGC AAG TGC AGG TCA CCT GAG CTG GAG
Ala Ser Asp Asp Leu Leu Gln Trp Pro Gln Ile Ser Lys Cys Arg Ser Pro Glu Leu Glu

                                   150                                   180
ACA TTT TCG TGT TAT TGG ACT GAT GGA AAC CTC ACT ACT TCA GGA ACA ATA CAA CTG TTG
Thr Phe Ser Cys Tyr Trp Thr Asp Gly Asn Leu Thr Thr Ser Gly Thr Ile Gln Leu Leu

                                   210                                   240
TAT ATG AAA AGG AGT GAT GAA GAC TGG AAA GAA TGT CCG GAT TAT ATC ACT GCA GGA GAA
Tyr Met Lys Arg Ser Asp Glu Asp Trp Lys Glu Cys Pro Asp Tyr Ile Thr Ala Gly Glu

                                   270                                   300
AAT AGC TGT TAC TTC AAC ACA TCC TAC ACC TCG ATT TGG ATA CCA TAT TGT GTT AAG CTT
Asn Ser Cys Tyr Phe Asn Thr Ser Tyr Thr Ser Ile Trp Ile Pro Tyr Cys Val Lys Leu

                                   330                                   360
GCC AAT AAA GAT GAA GTA TTT GAG GAA AAG TGT TTC AGT GTT GAT GAA ATA GTA CTA CCT
Ala Asn Lys Asp Glu Val Phe Asp Glu Lys Cys Phe Ser Val Asp Glu Ile Val Leu Pro

                                   390                                   420
GAT CCC CCT GTG CAC CTT AAC TGG ACT CTG CTA AAT ACT AGT CAA ACT GGG ATC CAT GGG
Asp Pro Pro Val His Leu Asn Trp Thr Leu Leu Asn Thr Ser Gln Thr Gly Ile His Gly

                                   450                                   480
GAT ATT CAA GTA CGA TGG GAT CCA CCA CCA ACA GCA GAT GTT CAG AAA GGA TGG ATT ACT
Asp Ile Gln Val Arg Trp Asp Pro Pro Pro Thr Ala Asp Val Gln Lys Gly Trp Ile Thr

                                   510                                   540
CTG GAG TAT GAA TTG CAG TAC AAA GAA GTT AAT GAG ACA AAA TGG AAG GAG CAT TTC TGT
Leu Glu Tyr Glu Leu Gln Tyr Lys Glu Val Asn Glu Thr Lys Trp Lys Glu His Phe Cys

CTT TGC AGT TAG
Leu Cys Ser End
```

FIG. 1

30 60
ATG GAT CTT CGG CAT CTG CTG TTT ACT TTG GCA CTG GTG TGT GCA AAT GAC TCA CTT TCT
Met Asp Leu Arg His Leu Leu Phe Thr Leu Ala Leu Val Cys Ala Asn Asp Ser Leu Ser

90 120
GCA AGT GAT GAT CTT CTG CAG TGG CCA CAA ATC AGC AAG TGC AGG TCA CCT GAG CTG GAG
Ala Ser Asp Asp Leu Leu Gln Trp Pro Gln Ile Ser Lys Cys Arg Ser Pro Glu Leu Glu

150 180
ACA TTT TCG TGT TAT TGG ACT GAT GGA AAC CTC ACT ACT TCA GGA ACA ATA CAA CTG TTG
Thr Phe Ser Cys Tyr Trp Thr Asp Gly Asn Leu Thr Thr Ser Gly Thr Ile Gln Leu Leu

210 240
TAT ATG AAA AGG AGT GAT GAA GAC TGG AAA GAA TGT CCG GAT TAT ATC ACT GCA GGA GAA
Tyr Met Lys Arg Ser Asp Glu Asp Trp Lys Glu Cys Pro Asp Tyr Ile Thr Ala Gly Glu

270 300
AAT AGC TGT TAC TTC AAC ACA TGG TAC ACC TCG ATT TGG ATA CCA TAT TGT GTT AAG CTT
Asn Ser Cys Tyr Phe Asn Thr Ser Tyr Thr Ser Ile Trp Ile Pro Tyr Cys Val Lys Leu

330 360
GCC AAT AAA GAT GAA GTA TTT GAC GAA AAG TGT TTC AGT GTT GAT GAA ATA GTA CTA CCT
Ala Asn Lys Asp Glu Val Phe Asp Glu Lys Cys Phe Ser Val Asp Glu Ile Val Leu Pro

390 420
GAT CCC CCT GTG CAC CTT AAC TGG ACT CTG CTA AAT ACT AGT CAA ACT GGG ATC CAT GGG
Asp Pro Pro Val His Leu Asn Trp Thr Leu Leu Asn Thr Ser Gln Thr Gly Ile His Gly

450 480
GAT ATT CAA GTA CGA TGG GAT CCA CCA CCA ACA GCA GAT GTT CAG AAA GGA TGG ATT ACT
Asp Ile Gln Val Arg Trp Asp Pro Pro Pro Thr Ala Asp Val Gln Lys Gly Trp Ile Thr

FIG.2A

510 540
CTG GAG TAT GAA TTG CAG TAC AAA GAA GTT AAT GAG ACA AAA TGG AAG GAG TTA GAA CCC
Leu Glu Tyr Glu Leu Gln Tyr Lys Glu Val Asn Glu Thr Lys Trp Lys Glu Leu Glu Pro

570 600
AGG CTC TCA ACA GTG GTT CCA CTG TAT TCT CTG AAG ATG GAA GAG ATT ATG AGA TCC GAG
Arg Leu Ser Thr Val Val Pro Leu Tyr Ser Leu Lys Met Glu Glu Ile Met Arg Ser Glu

630 660
TCC GAT CAA GAC AAC GTA CCT CCG AAA AGT TTG GGG AGT TCA GTG AAA TCC TCT ATG TTT
Ser Asp Gln Asp Asn Val Pro Pro Lys Ser Leu Gly Ser Ser Val Lys Ser Ser Met Phe

690 720
CCT TTA CTC AAG CAG GCA TTG AAT TTG TTC ATT GTG CTG AAG AAA TCG AGT TTC CCT GGT
Pro Leu Leu Lys Gln Ala Leu Asn Leu Phe Ile Val Leu Lys Lys Ser Ser Phe Pro Gly

TCT TAG
Ser End

FIG.2B

```
                                             30                                      60
ATG GAT CTT CGG CAT CTG CTG TTT ACT TTG GCA CTG GTG TGT GCA AAT GAC TCA CTT TCT
Met Asp Leu Arg His Leu Leu Phe Thr Leu Ala Leu Val Cys Ala Asn Asp Ser Leu Ser

                                             90                                     120
GCA AGT GAT GAT CTT CTG CAG TGG CCA CAA ATC AGC AAG TGC AGG TCA CCT GAG CTG GAG
Ala Ser Asp Asp Leu Leu Gln Trp Pro Gln Ile Ser Lys Cys Arg Ser Pro Glu Leu Glu

                                            150                                     180
ACA TTT TCG TGT TAT TGG ACT GAT GGA AAC CTC ACT ACT TCA GGA ACA ATA CAA CTG TTG
Thr Phe Ser Cys Tyr Trp Thr Asp Gly Asn Leu Thr Thr Ser Gly Thr Ile Gln Leu Leu

                                            210                                     240
TAT ATG AAA AGG AGT GAT GAA GAC TGG AAA GAA TGT CCG GAT TAT ATC ACT GCA GGA GAA
Tyr Met Lys Arg Ser Asp Glu Asp Trp Lys Glu Cys Pro Asp Tyr Ile Thr Ala Gly Glu

                                            270                                     300
AAT AGC TGT TAC TTC AAC ACA TCC TAC ACC TCG ATT TGG ATA CCA TAT TGT GTT AAG CTT
Asn Ser Cys Tyr Phe Asn Thr Ser Tyr Thr Ser Ile Trp Ile Pro Tyr Cys Val Lys Leu

                                            330                                     360
GCC AAT AAA GAT GAA GTA TTT GAC GAA AAG TGT TTC AGT GTT GAT GAA ATA GTA CTA CCT
Ala Asn Lys Asp Glu Val Phe Asp Glu Lys Cys Phe Ser Val Asp Glu Ile Val Leu Pro

                                            390                                     420
GAT CCC CCT GTG CAC CTT AAC TGG ACT CTG CTA AAT ACT AGT CAA ACT GGG ATC CAT GGG
Asp Pro Pro Val His Leu Asn Trp Thr Leu Leu Asn Thr Ser Gln Thr Gly Ile His Gly
```

FIG.3A

```
                                            450                                     480
GAT ATT CAA GTA CGA TGG GAT CCA CCA CCA ACA GCA GAT GTT CAG AAA GGA TGG ATT ACT
Asp Ile Gln Val Arg Trp Asp Pro Pro Pro Thr Ala Asp Val Gln Lys Gly Trp Ile Thr

                                            510                                     540
CTG GAG TAT GAA TTG CAG TAC AAA GAA GTT AAT GAG ACA AAA TGG AAG GAG TTA GAA CCC
Leu Glu Tyr Glu Leu Gln Tyr Lys Glu Val Asn Glu Thr Lys Trp Lys Glu Leu Glu Pro

                                            570                                     600
AGG CTC TCA ACA GTG GTT CCA CTG TAT TCT CTG AAG ATG GGA AGA GAT TAT GAG ATC CGA
Arg Leu Ser Thr Val Val Pro Leu Tyr Ser Leu Lys Met Gly Arg Asp Tyr Glu Ile Arg

                                            630                                     660
GTC CGA TCA AGA CAA CGT ACC TCC GAA AAG TTT GGG GAG TTC AGT GAA ATC CTC TAT GTT
Val Arg Ser Arg Gln Arg Thr Ser Glu Lys Phe Gly Glu Phe Ser Glu Ile Leu Tyr Val

                                            690                                     720
TCC TTT ACT CAA GCA GGC ATT GAA TTT GTT CAT TGT GCT GAA GAA ATC GAG TTT CCC TGG
Ser Phe Thr Gln Ala Gly Ile Glu Phe Val His Cys Ala Glu Glu Ile Glu Phe Pro Trp

                                            750                                     780
TTC TTA GTT GTT GTC TTC GGA GTG TGT GGG CTG GCC GTA ACA GCG ATC TTA ATC CTG TTG
Phe Leu Val Val Val Phe Gly Val Cys Gly Leu Ala Val Thr Ala Ile Leu Ile Leu Leu

                                            810                                     840
TCT AAA CAG CCA AGG TTA AAA ATG CTG ATT TTT CCT CCT GTG CCA GTT CCA AAG ATT AAA
Ser Lys Gln Pro Arg Leu Lys Met Leu Ile Phe Pro Pro Val Pro Val Pro Lys Ile Lys
```

FIG. 3B

```
                                    870                                    900
GGG ATT GAC CCA GAT CTC TTA AAG AAA GGA AAG CTA GAT GAA GTG AAC TCC ATC TTA GCC
Gly Ile Asp Pro Asp Leu Leu Lys Lys Gly Lys Leu Asp Glu Val Asn Ser Ile Leu Ala

                                    930                                    960
AGC CAT GAC AAC TAC AAG ACA CAG CTA TAC AAT GAT GAC TTG TGG GTT GAG TTT ATT GAA
Ser His Asp Asn Tyr Lys Thr Gln Leu Tyr Asn Asp Asp Leu Trp Val Glu Phe Ile Glu

                                    990                                   1020
TTG GAC ATA GAT GAC TCC GAT GAA AAG AAC AGA GTC TCA GAT ACT GAC AGG CTC CTG AGT
Leu Asp Ile Asp Asp Ser Asp Glu Lys Asn Arg Val Ser Asp Thr Asp Arg Leu Leu Ser

                                   1050                                   1080
GAC GAT CAT CTG AAG TCA CAC AGT TGC TTG GGA GCC AAG GAT GAT GAT TCT GGA CGT GCC
Asp Asp His Leu Lys Ser His Ser Cys Leu Gly Ala Lys Asp Asp Asp Ser Gly Arg Ala

                                   1110                                   1140
AGT TGT TAT GAA CCA GAT ATT CCA GAG ACA GAC TTC AGT GCA AGC GAC ACA TGT GAT GCC
Ser Cys Tyr Glu Pro Asp Ile Pro Glu Thr Asp Phe Ser Ala Ser Asp Thr Cys Asp Ala

                                   1170                                   1200
ATC TCT GAT ATT GAT CAG TTC AAG AAG GTA ACT GAA AAA GAA GAG GAT CTC TTG TGC CTT
Ile Ser Asp Ile Asp Gln Phe Lys Lys Val Thr Glu Lys Glu Glu Asp Leu Leu Cys Leu

                                   1230                                   1260
CAT AGG AAA GAT GAT GTT GAG GCA CTT CAA AGT CTT GCC AAC ACA GAT ACC CAA CAG CCG
His Arg Lys Asp Asp Val Glu Ala Leu Gln Ser Leu Ala Asn Thr Asp Thr Gln Gln Pro
```

FIG. 3C

```
                                        1290                                        1320
CAT ACT AGT ACT CAG TCT GAA AGC AGA GAG TCA TGG CCA CCT TTT GCA GAC AGC ACT GAC
His Thr Ser Thr Gln Ser Glu Ser Arg Glu Ser Trp Pro Pro Phe Ala Asp Ser Thr Asp

                                        1350                                        1380
TCA GCT AAT CCA TCA GTC CAA ACT CAG CTA AGT AAC CAG AAT TCC CTG ACA AAC ACT GAC
Ser Ala Asn Pro Ser Val Gln Thr Gln Leu Ser Asn Gln Asn Ser Leu Thr Asn Thr Asp

                                        1410                                        1440
TTC TAT GCT CAA GTG AGT GAT ATT ACT CCT GCT GGA AGT GTT GTA CTT TCT CCA GGG CAG
Phe Tyr Ala Gln Val Ser Asp Ile Thr Pro Ala Gly Ser Val Val Leu Ser Pro Gly Gln

                                        1470                                        1500
AAA TCC AAG GTG GGA AGA GCA CAG TGT GAA AGC TGC ACA GAA CAA AAC TTC ACC ATG GAC
Lys Ser Lys Val Gly Arg Ala Gln Cys Glu Ser Cys Thr Glu Gln Asn Phe Thr Met Asp

                                        1530                                        1560
AAT GCC TAT TTC TGT GAG GCA GAT GTG AAA AAA TGT ATT GCT GTG ATT TCA CAG GAA GAG
Asn Ala Tyr Phe Cys Glu Ala Asp Val Lys Lys Cys Ile Ala Val Ile Ser Gln Glu Glu
```

FIG. 3D

```
                                    1590                                1620
AAT GAG CCG CGT GTT CAG GAG CAA AGC TGT AAC GAG GAC ACT TAC TTC ACC ACA GAA AGC
Asn Glu Pro Arg Val Gln Glu Gln Ser Cys Asn Glu Asp Thr Tyr Phe Thr Thr Glu Ser

                                    1650                                1680
CTT ACC ACT ACC GGT ATC AAT CTT GGA GCT TCA ATG GCA GAA ACC CCA AGT ATG GAA ATG
Leu Thr Thr Thr Gly Ile Asn Leu Gly Ala Ser Met Ala Glu Thr Pro Ser Met Glu Met

                                    1710                                1740
CCT GTC CCA GAC TAC ACT TCT ATT CAT ATT GTT CAC TCT CCA CAA GGC CTT GTG CTC AAT
Pro Val Pro Asp Tyr Thr Ser Ile His Ile Val His Ser Pro Gln Gly Leu Val Leu Asn

                                    1770                                1800
GCA ACT GCA CTG CCT GTG CCA GAG AAA GAA TTT AAC ATG TCT TGT GGC TAT GTG AGC ACA
Ala Thr Ala Leu Pro Val Pro Glu Lys Glu Phe Asn Met Ser Cys Gly Tyr Val Ser Thr

GAC CAG CTG AAC AAA ATC ATG CCG TAG
Asp Gln Leu Asn Lys Ile Met Pro End
```

FIG. 3E

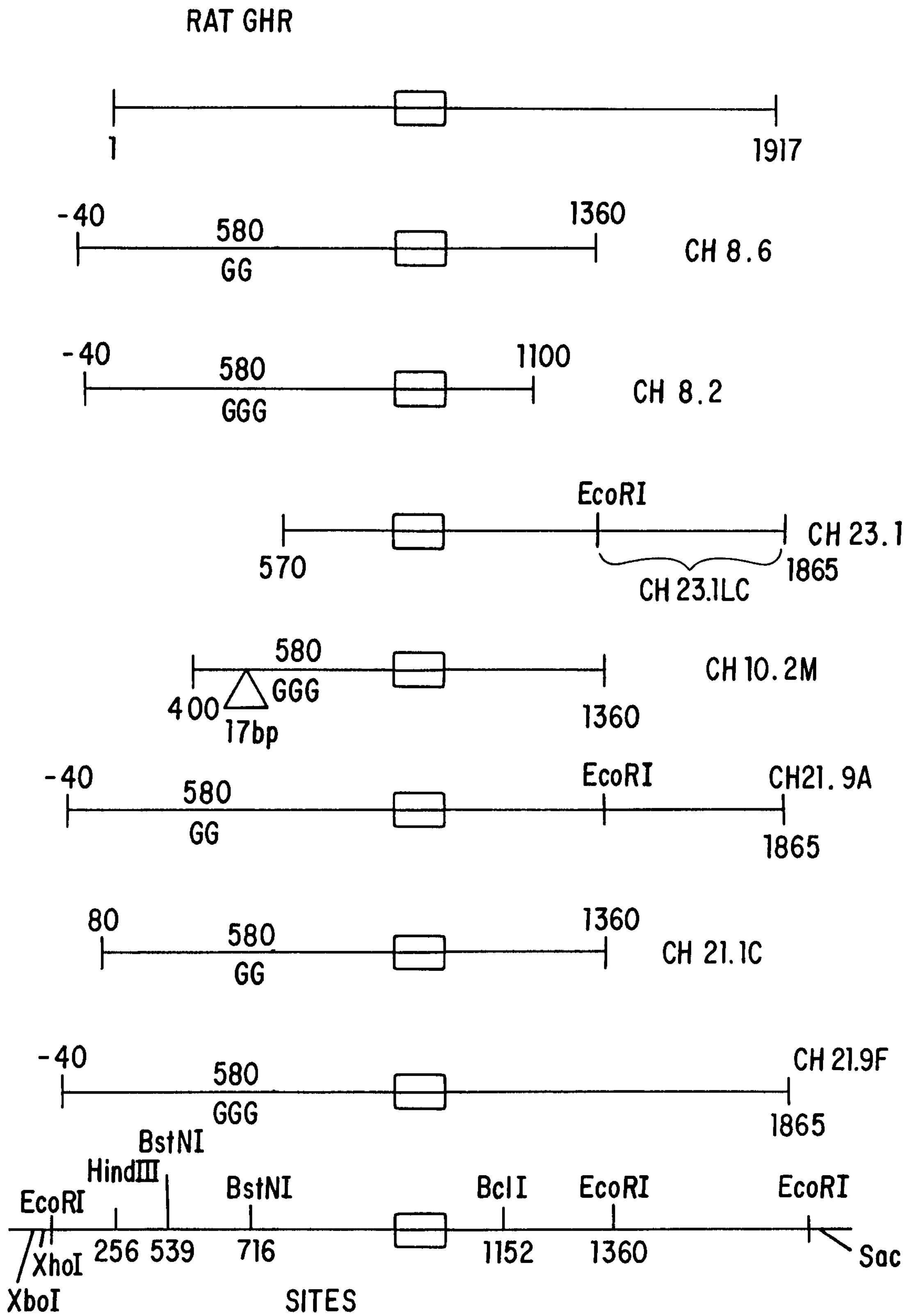
RAT GHR
1
1917
-40
580
GG
1360
CH 8.6
-40
580
GGG
1100
CH 8.2
EcoRI
570
CH 23.1
1865
CH 23.1LC
580
GGG
400
17bp
1360
CH 10.2M
-40
580
GG
EcoRI
CH21.9A
1865
80
580
GG
1360
CH 21.1C
-40
580
GGG
CH 21.9F
1865
BstNI
HindIII
EcoRI
BstNI
BclI
EcoRI
EcoRI
XhoI
256
539
716
1152
1360
Sac
XboI
SITES

FIG. 4

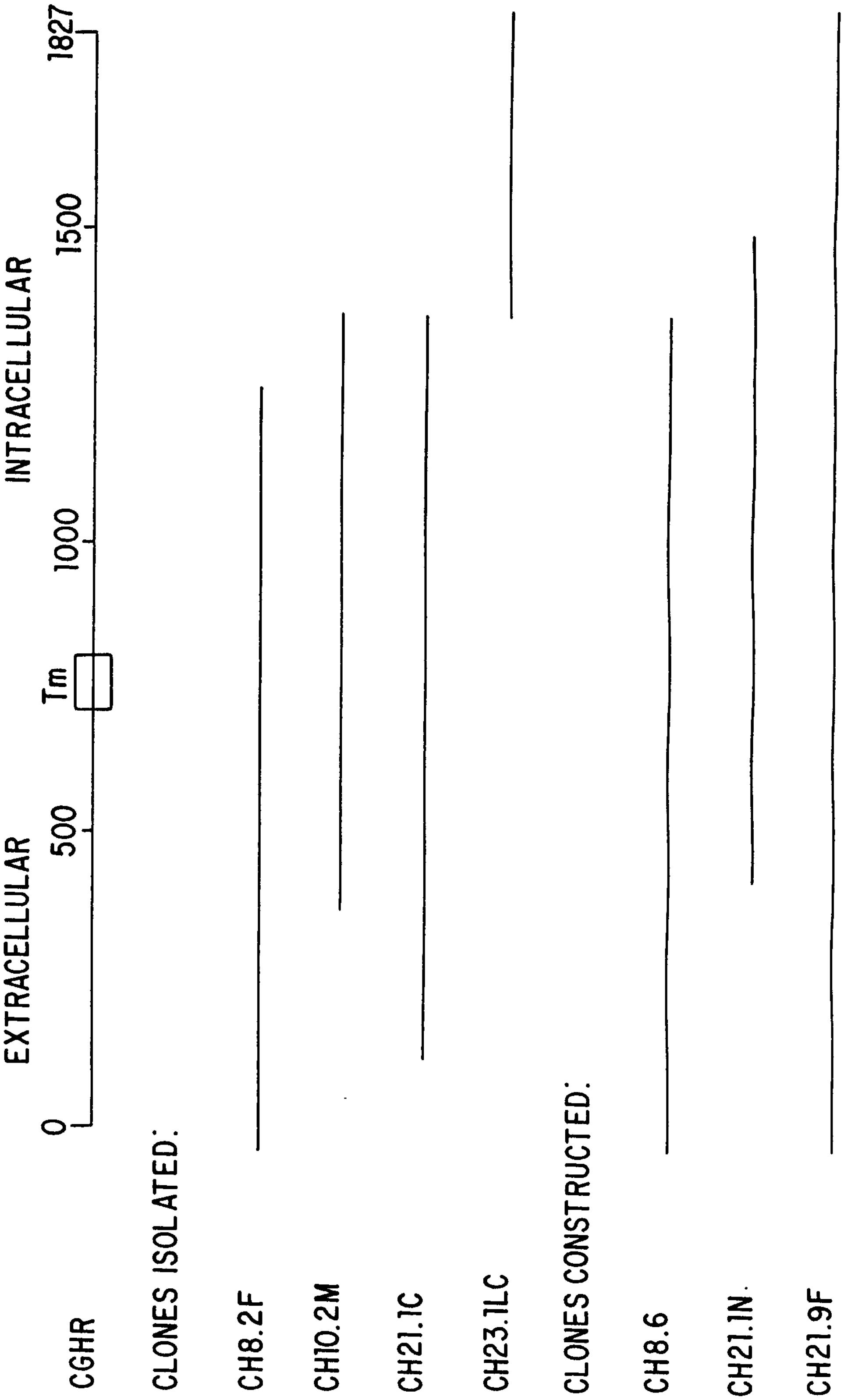
CGHR
CLONES ISOLATED:
CH8.2F
CH10.2M
CH21.1C
CH23.1LC
CLONES CONSTRUCTED:
CH8.6
CH21.1N
CH21.9F
0
500
1000
1500
1827
Tm
EXTRACELLULAR
INTRACELLULAR

FIG. 5

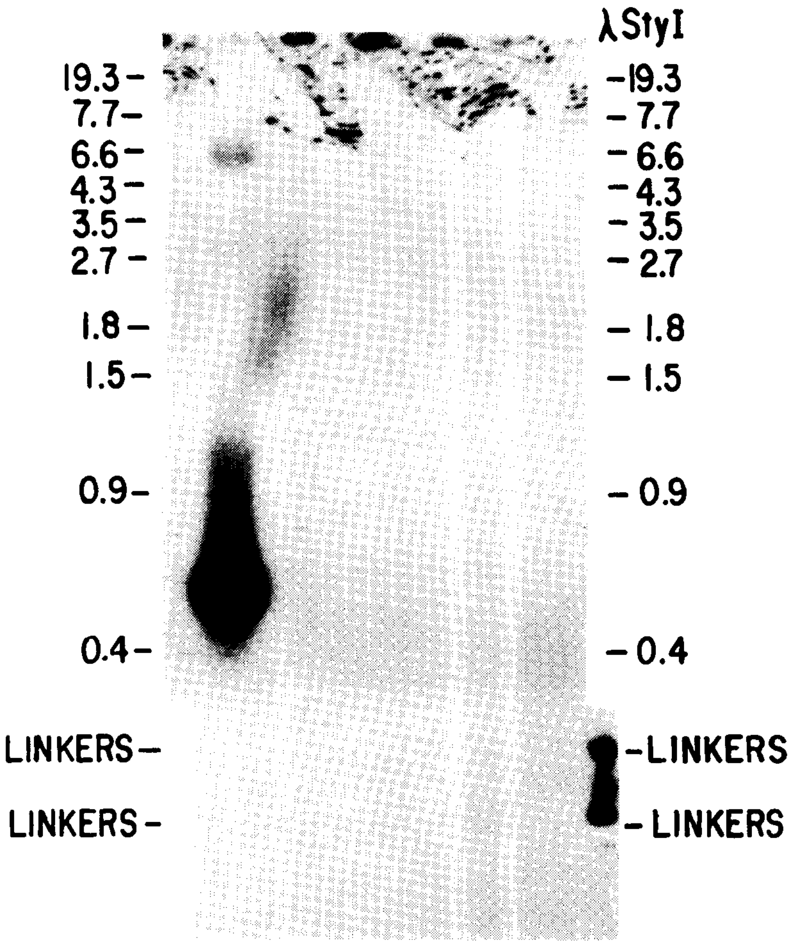
λStyI
19.3
7.7
6.6
4.3
3.5
2.7
1.8
1.5
0.9
0.4
LINKERS
LINKERS
19.3
7.7
6.6
4.3
3.5
2.7
1.8
1.5
0.9
0.4
LINKERS
LINKERS

FIG. 6A

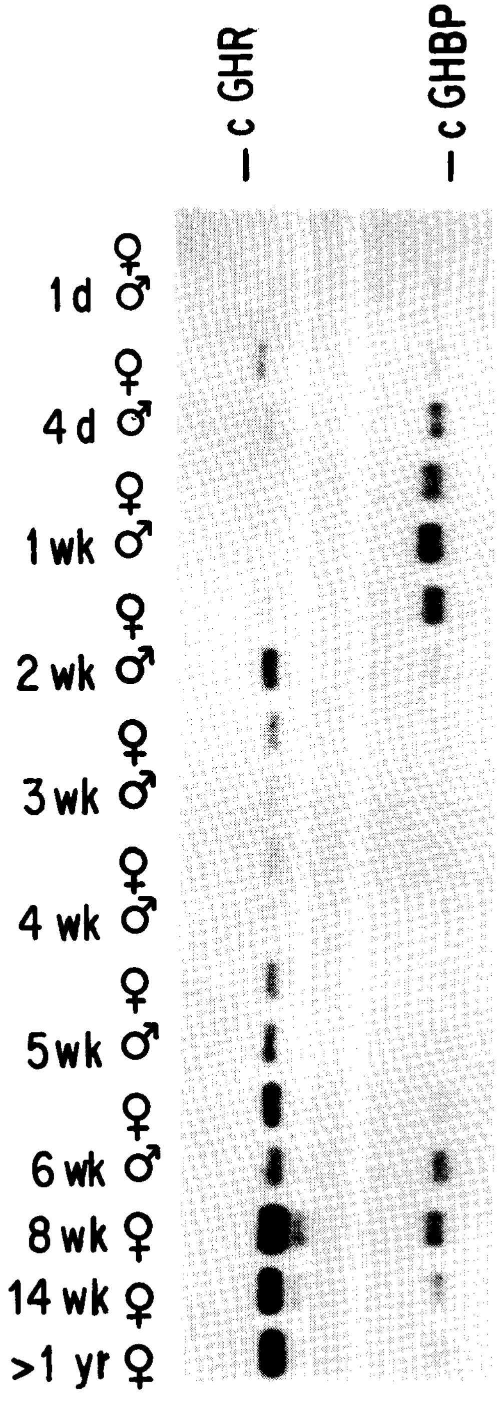


FIG. 6B

```
-178
  GCGGCTGA CGGCCAGCGA GCTGCTGAGC CCCGCCGCCC CCGGGCAGGG GCAGCGCGGA

-120
GGGGGCTGCC GGCTCCCGCG AGCGAAGTGC TGGGATGGCT GGAGAGGGCC GCATTTTGCA

-60
GTTGAGCTGA GGACGTGTGA AGCATCATCC ACCCCCTGCT GACATTTGAG AATTAGAGGT

        10         20         30         40         50         60
ATGGATCTTC GGCATCTGCT GTTTACTTTG GCACTGGTGT GTGCAAATGA CTCACTTTCT

        70         80         90        100        110        120
GCAAGTGATG ATCTTCTGCA GTGGCCACAA ATCAGCAAGT GCAGGTCACC TGAGCTGGAG

       130        140        150        160        170        180
ACATTTTCGT GTTATTGGAC TGATGGAAAC CTCACTACTT CAGGAACAAT ACAACTGTTG

       190        200        210        220        230        240
TATATGAAAA GGAGTGATGA AGACTGGAAA GAATGTCCGG ATTATATCAC TGCAGGAGAA

       250        260        270        280        290        300
AATAGCTGTT ACTTCAACAC ATCCTACACC TCGATTTGGA TACCATATTG TGTTAAGCTT

       310        320        330
GCCAATAAAG ATGAAGTATT TGACGAAAAA AA
```

FIG.7

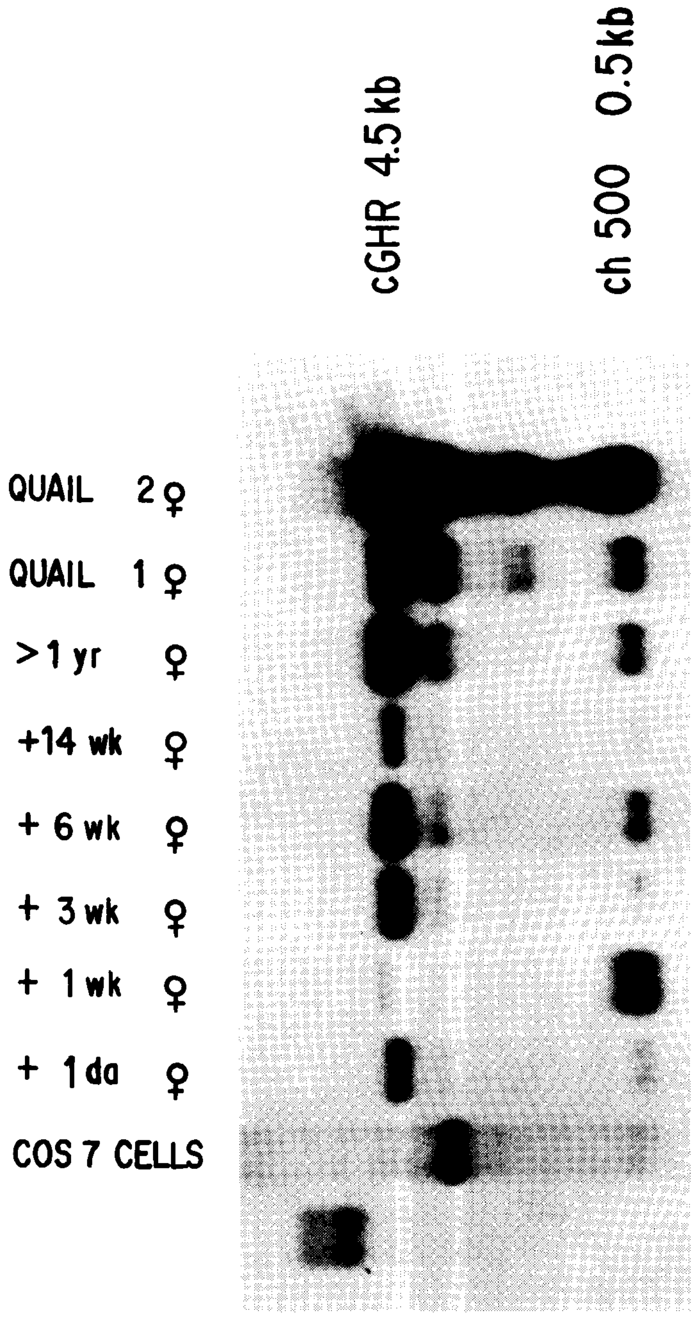
cGHR 4.5 kb
ch 500 0.5kb
QUAIL 2 ♀
QUAIL 1 ♀
>1 yr ♀
+14 wk ♀
+ 6 wk ♀
+ 3 wk ♀
+ 1 wk ♀
+ 1 da ♀
COS 7 CELLS

FIG. 8

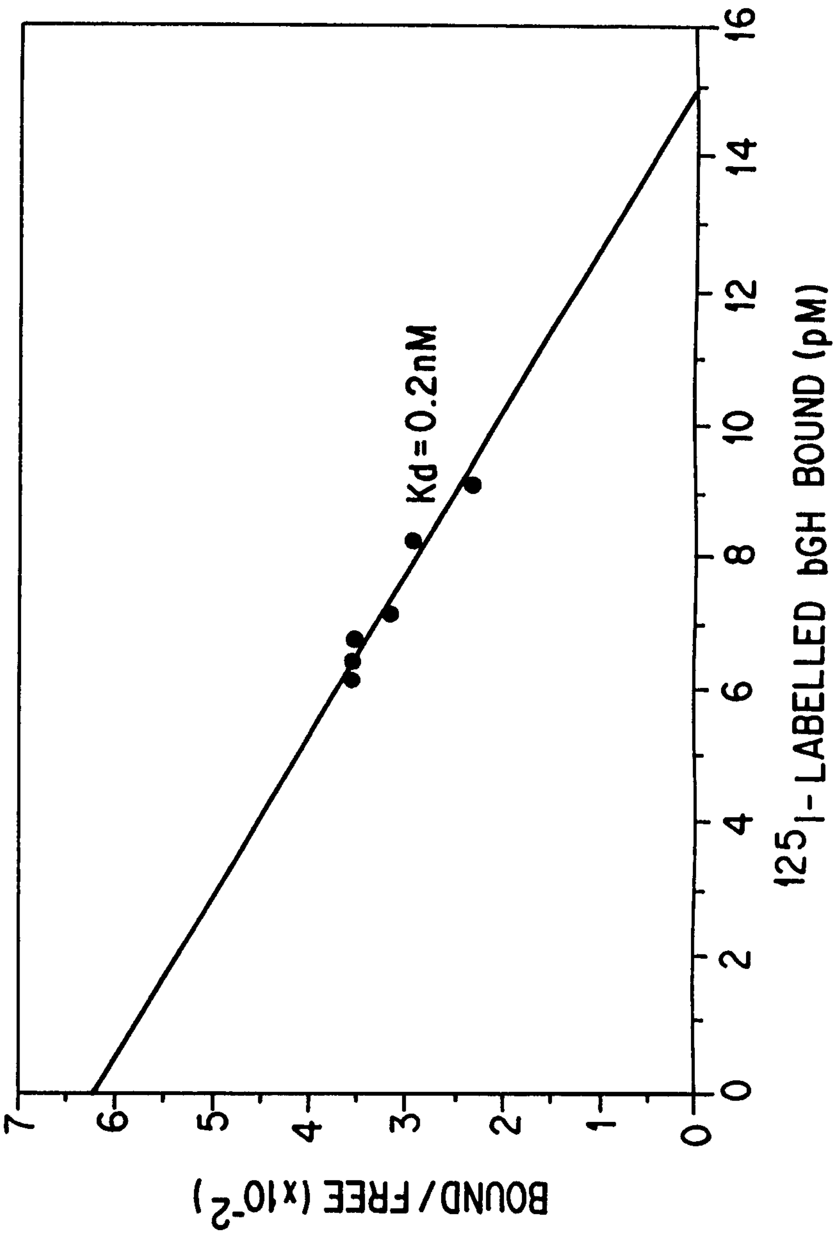
BOUND/ FREE (x10^-2)
0
1
2
3
4
5
6
7
0
2
4
6
8
10
12
14
16
Kd = 0.2nM
125I- LABELLED bGH BOUND (pM)

FIG. 9